# EUROPEAN PATENT SPECIFICATION

(11) **EP 4 277 885 B1**
(45) Date of publication and mention of the grant of the patent: **26.02.2025**
(21) Application number: 22701332.3
(22) Date of filing: 13.01.2022
(51) Int. Cl.: C07C 29/17, C07C 33/26

(54) **PROCESS FOR THE PREPARATION OF A CHIRAL TRIOL**
VERFAHREN ZUR HERSTELLUNG VON CHIRALEN TRIOLEN
PROCÉDÉ DE PRÉPARATION D'UN TRIOL CHIRAL

(30) Priority: 15.01.2021 EP 21151758
(43) Date of publication of application: 22.11.2023
(73) Proprietor: F. Hoffmann-La Roche AG, 4070 Basel (CH); Genentech, Inc., South San Francisco, CA 94080 (US)
(72) Inventor: GLASS, Anna-Lena, 4070 Basel (CH); HONG, Allen Yu, San Francisco, California 94080 (US); PUENTENER, Kurt, 4070 Basel (CH)
(74) Representative: Rauber, Beat
(86) International application number: PCT/EP2022/050575
(87) International publication number: WO 2022/152769

(56) References cited:
- XIAO-HUI YANG ET AL: "Catalytic Asymmetric Hydrogenation of [delta]-Ketoesters: Highly Efficient Approach to Chiral 1,5-Diols", ANGEWANDTE CHEMIE INTERNATIONAL EDITION, vol. 52, no. 30, 22 July 2013 (2013-07-22), pages 7833 - 7836, XP055098608, ISSN: 1433-7851, DOI: 10.1002/anie.201303011
- TICOZZI CALIMERO ET AL: "Synthesis of a bifunctional 2,4-dihydroxy five-carbon synthon. Enantiomerically pure [Delta]2-isoxazolines by chromatographic resolution", TETRAHEDRON LETTERS, vol. 35, no. 40, 1 October 1994 (1994-10-01), Amsterdam , NL, pages 7421 - 7424, XP055814517, ISSN: 0040-4039, DOI: 10.1016/0040-4039(94)85331-2

## Description

The invention relates to a process for the preparation of the chiral triol of the formula I 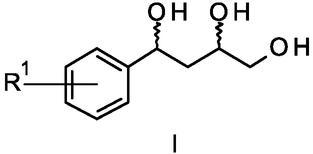 wherein
R¹ is hydrogen or halogen and
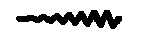 denotes either a dashed bond (a) or a wedged bond (b)
   a) 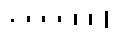 b) 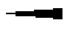 .

Chiral triols are versatile building blocks for the preparation of various pharmaceutically active drug substances such as for instance for statin drugs (A. Lenhart, W.D. Chey "Adv. Nutr. 2017, 8(4), 587-596).

C.Ticozzi, A.Zanarotti, Tetrahedron Letters, 1994, 35(40), 7421-7424 describe a synthesis of chiral triols by way of the diastereoselective reduction of a hydroxy ketone precursor (S)-2 or (R)-2 with borohydride type catalysts.

The object of the present invention was to provide a process which allows the preparation of the chiral triol in a scalable manner with high enantiomeric purity and yield.

The object could be reached with the process for the preparation of the chiral triol of formula I 3651 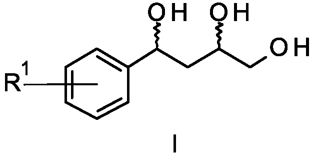 wherein
R¹ is hydrogen or halogen and
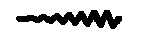 denotes either a dashed bond (a) or a wedged bond (b)
   a) 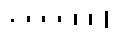 b) 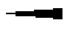 .

and which comprises the asymmetric hydrogenation of a ketone compound of formula IIa 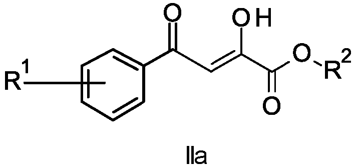 3651
   wherein
   R¹ is hydrogen or halogen and
   R² is C₁₋₆-alkyl;
with hydrogen in the presence of an iridium spiro-pyridylamidophosphine catalyst (Ir-SpiroPAP catalyst) of the formula IIIa or IIIb, or enantiomers thereof 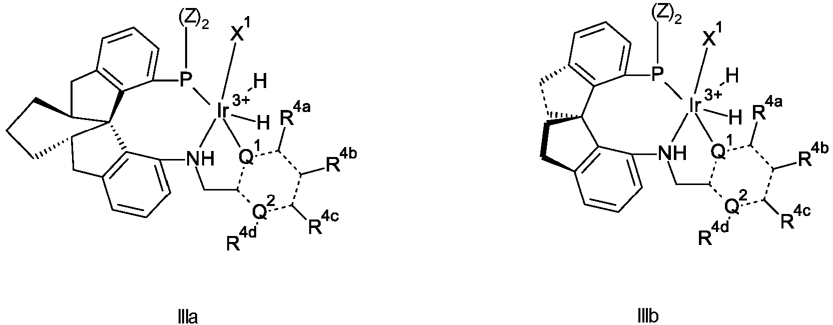 wherein
   R^{4a}, R^{4b}, R^{4c} and R^{4d} independently of each other are hydrogen or C₁₋₆-alkyl;
   the dotted ring signifies an aromatic ring when Q¹ is nitrogen and Q² is carbon and the dotted ring signifies a cycloalkane ring wherein Q¹ and Q² are sulfur;
   X¹ is either a coordinated ligand or a counter anion selected from halogen, C₁₋₆-alkoxy, tetrahalogenoborate, hexahalogenoborate, tetrakis (3,5-bis(trihalogeno-C₁₋₆-alkyl) phenyl)borate, acetylacetonate, hexahalogenophosphate, p-tolylsulfonate (OTs) or trihalogeno methanesulfonate and
   Z is phenyl, optionally substituted by one or more groups selected from
   C₁₋₈-alkyl, C₁₋₈-halogenalkyl or phenyl; C₃₋₈-cycloalkyl, optionally substituted by one or more C₁₋₈-alkyl groups or di- C₁₋₈-alkyl phosphinyl.

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below.

The term "chiral" denotes the ability of non-superimposability with the mirror image, while the term "achiral" refers to embodiments which are superimposable with their mirror image. Chiral molecules are optically active, i.e., they have the ability to rotate the plane of plane-polarized light. Whenever a chiral center is present in a chemical structure, it is intended that all stereoisomers associated with that chiral center are encompassed by the present invention.

The term "chiral" signifies that the molecule can exist in the form of optically pure enantiomers, mixtures of enantiomers, optically pure diastereoisomers or mixtures of diastereoisomers.

In a preferred embodiment of the invention the term "chiral" denotes optically pure enantiomers or optically pure diastereoisomers.

The term "stereoisomer" denotes a compound that possesses identical molecular connectivity and bond multiplicity, but which differs in the arrangement of its atoms in space.

The term "diastereomer" denotes a stereoisomer with two or more centers of chirality and whose molecules are not mirror images of one another. Diastereomers may have different physical properties, e.g. melting points, boiling points, spectral properties, and reactivities.

The term "enantiomers" denotes two stereoisomers of a compound which are non-superimposable mirror images of one another.

In the structural formula presented herein a dashed bond (a) denotes that the substituent is below the plane of the paper and a wedged bond (b) denotes that the substituent is above the plane of the paper.
a) 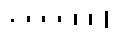 b) 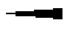 .

The spiral bond (c) denotes both options i.e. either a dashed bond (a) or a wedged bond (b).
c) 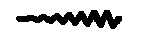

The term "C-₁₋₈- alkyl" denotes a monovalent linear or branched saturated hydrocarbon group of 1 to 8 carbon atoms. Examples of C₁₋₈-alkyl include methyl, ethyl, propyl, isopropyl, n-butyl, isobutyl, sec-butyl, tert-butyl or pentyl, hexyl, heptyl or octyl with its isomers. Preferably the term denotes a C-₁₋₆- alkyl group.

The term "C₃₋₈-cycloalkyl" denotes a saturated carbocycle of 3 to 8 carbon atoms. Examples of C₃₋₈-cycloalkyl include cyclopropyl, cyclobutyl, cyclopentyl, cyclohexyl, cycloheptyl and cyclooctyl with its isomers. Preferably the term encompasses C₄₋₇-cycloalkyl, more preferably cyclpentyl and cyclohexyl.

The term "C-₁₋₆- alkoxy" denotes a monovalent linear or branched saturated hydrocarbon group of 1 to 6 carbon atoms attached to an oxygen atom. Examples of C₁₋₆-alkoxy include methoxy, ethoxy, propoxy, isopropoxy, n-butoxy, iso-butoxy, sec-butoxy, tert-butoxy, or pentoxy or hexoxy

with its isomers. Preferably the term denotes a C-₁₋₄- alkoxy group, more preferably the methoxy group.

The term "halogen" denotes fluoro, chloro, bromo, or iodo.

The term "C-₁₋₈- halogenalkyl" denotes a monovalent linear or branched saturated hydrocarbon group of 1 to 8 carbon atoms which is substituted by one or more halogen atoms. Preferably the term denotes C-₁₋₄- halogenalkyl, more preferably a methyl group which is substituted with one or more halogen atoms such as trifluoromethyl.

The ketone of formula IIa may occur in the mesomeric structures outlined in the scheme below. For the sake of clarity the formula IIa is consistently used throughout this description.

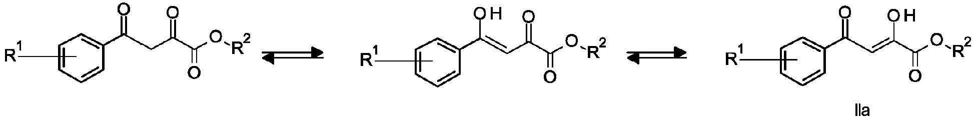

The process of the present invention can be illustrated with the scheme 1 below 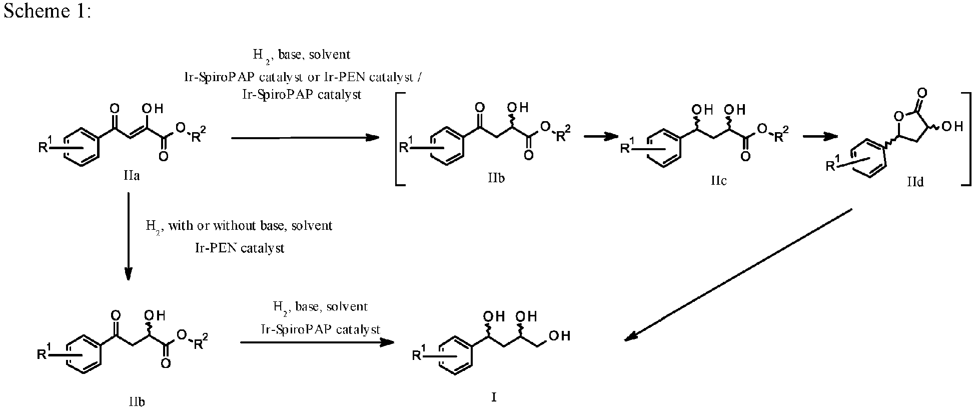 and comprises the following various principal embodiments for the preparation of the chiral triol of formula I.
a) The asymmetric hydrogenation of the ketone of formula IIa in the sole presence of an Ir-SpiroPAP catalyst.
b) The asymmetric hydrogenation of the ketone of formula IIa in the presence of an in situ formed Ir-SpiroPAP catalyst.
c) The asymmetric hydrogenation of the ketone of formula IIa in the sole presence of an Ir-PEN catalyst to form the ketone of formula IIb and its subsequent asymmetric hydrogenation to the chiral triol of formula I in the presence of the Ir-SpiroPAP catalyst.
d) The asymmetric hydrogenation of the ketone of formula IIa in the presence of a mixture of an Ir-SpiroPAP catalyst and an Ir-PEN catalyst.
e) The asymmetric hydrogenation of either intermediate IIb, IIc or IId in presence of an Ir-SpiroPAP catalyst.

The embodiments a) to d) are preferred, more preferred are the embodiments a), b) and d) and embodiment d) is most preferred.

In a preferred embodiment of the present invention the chiral triol has the formula Ia 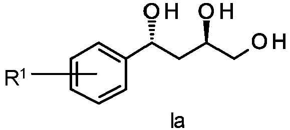 wherein R¹ is as above, but preferably stands for halogen, more preferably for chlorine.

R¹ can be in the ortho-, meta- or para-position of the phenyl ring, but preferably R¹ is in the para-position of the phenyl ring.

In a further preferred embodiment of the present invention the chiral triol has the formula Ib

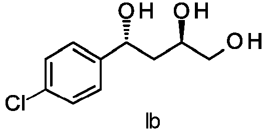

Scheme 2 illustrates a preferred embodiment of the invention.

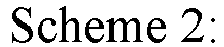

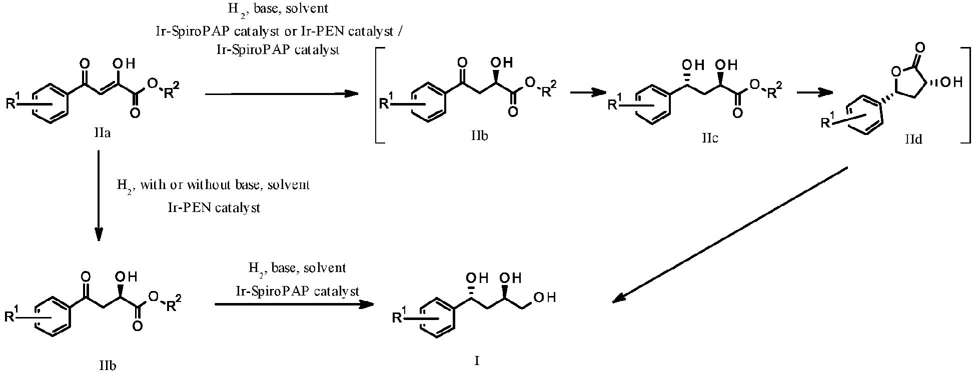

### a) The asymmetric hydrogenation of the ketone of formula IIa in the sole presence of an Ir-SpiroPAP catalyst

The iridium spiro-pyridylamidophosphine catalyst (Ir-SpiroPAP catalyst) are of the formula IIIa or IIIb, or enantiomers thereof 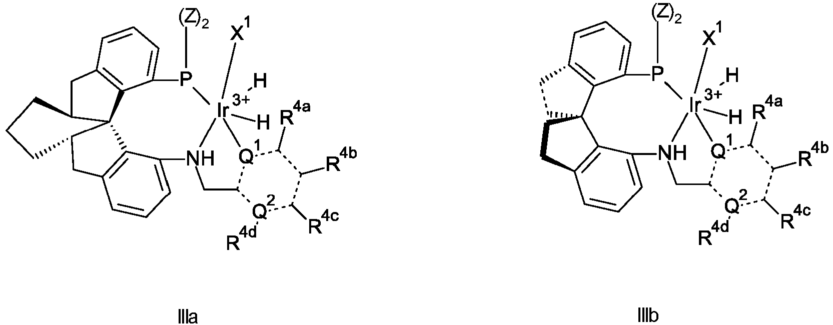 wherein
R^{4a}, R^{4b}, R^{4c} and R^{4d} independently of each other are hydrogen or C₁₋₆-alkyl;
the dotted ring signifies an aromatic ring when Q¹ is nitrogen and Q² is carbon and the dotted ring signifies a cycloalkane ring wherein Q¹ and Q² are sulfur;
X¹ is either a coordinated ligand or a counter anion selected from halogen, C₁₋₆-alkoxy, tetrahalogenoborate, hexahalogenoborate, tetrakis (3,5-bis(trihalogeno-C₁₋₆-alkyl)phenyl)borate, acetylacetonate, hexahalogenophosphate, p-tolylsulfonate (OTs) or trihalogeno methanesulfonate and
Z is phenyl, optionally substituted by one or more groups selected from C₁₋₈-alkyl, C₁₋₈-halogenalkyl or phenyl; C₃₋₈-cycloalkyl, optionally substituted by one or more C₁₋₈-alkyl groups or di- C₁₋₈-alkyl phosphinyl.

In a preferred embodiment
R^{4a}, R^{4b}, R^{4c} and R^{4d} independently of each other are hydrogen or C₁₋₄-alkyl;
the dotted ring signifies an aromatic ring when Q¹ is nitrogen and Q² is carbon and the dotted ring signifies a cycloalkane ring wherein Q¹ and Q² are sulfur;
X¹ is either a coordinated ligand or a counter anion selected from halogen, methoxy, tetrafluoroborate (BF4), hexafluoroborate (BF6), tetrakis(3,5-bis(trifluoromethyl) phenyl)borate (barf), acetylacetonate (acac), hexafluorophosphate (PF6), p-tolylsulfonate (OTs) or trifluoromethanesulfonate (OTf) and;
Z is phenyl, optionally substituted by one or more groups selected from C₁₋₆-alkyl, C₁₋₄-halogenalkyl or phenyl or is C₄₋₇-cycloalkyl.

In a further preferred embodiment
R^{4a}, R^{4b}, R^{4c} and R^{4d} independently of each other are hydrogen or C₁₋₄-alkyl;
the dotted ring signifies an aromatic ring when Q¹ is nitrogen and Q² is carbon and the dotted ring signifies a cycloalkane ring wherein Q¹ and Q² are sulfur;
X¹ is halogen;
Z is phenyl, optionally substituted by one or two groups selected from C₁₋₆-alkyl, C₁₋₄-halogenalkyl or phenyl or is cyclopentyl or cyclohexyl.

In a further preferred embodiment the iridium spiro-pyridylamidophosphine catalyst (Ir-SpiroPAP catalyst) are selected from the compounds 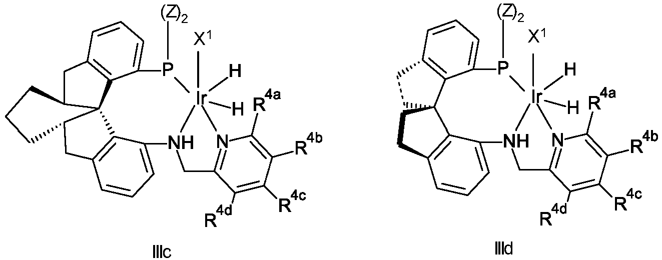 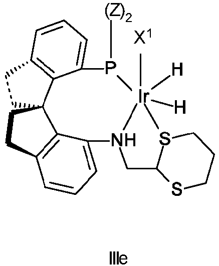 wherein;
R^{4a}, R^{4b}, R^{4c} and R^{4d} independently of each other are hydrogen or C₁₋₄-alkyl;
X¹ is halogen;
Z is phenyl optionally substituted by one or two groups selected from C₁₋₆-alkyl, C₁₋₄halogenalkyl or phenyl or is cyclopentyl or cyclohexyl.

In a further preferred embodiment the iridium spiro-pyridylamidophosphine catalyst (Ir-SpiroPAP catalyst) is selected from the compound 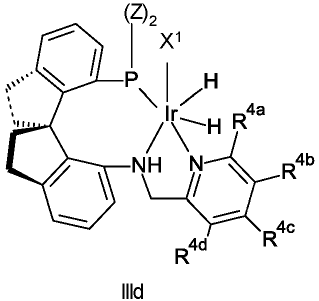 wherein
R^{4a}, R^{4b}, R^{4c} and R^{4d} independently of each other are hydrogen or C₁₋₄-alkyl;
X¹ is a ligand selected from halogen;
Z is phenyl, optionally substituted by one or two groups selected from C₁₋₆-alkyl, C₁₋₄-halogenalkyl or phenyl or is cyclopentyl or cyclohexyl.,
more preferably,
R^{4a}, R^{4b}, R^{4c} is hydrogen and R^{4d} is methyl;
X¹ is chlorine and
Z is phenyl, 3,5-dimetylphenyl, 3,5-di-tert-butyl phenyl, 3,5-di-tert-pentyl phenyl, 3,5-diphenyl phenyl, 4-phenyl phenyl, 3,5-di-trifluoromethyl phenyl, cyclohexyl or cyclopentyl.

Suitable catalysts are typically commercially available e.g. from Jiuzhou Pharma in China.

The asymmetric hydrogenation can be performed in the presence of suitable organic solvent and a base at a hydrogen pressure of 5 bar to 100 bar, preferably of 30 bar to 70 bar and at a reaction temperature of 10°C to 90°C, preferably of 20°C to 40°C.

The organic solvent can be selected from aliphatic alcohols selected from methanol, ethanol, isopropanol, tert-amylalcohol, from halogen substituted alcohols like trifluoroethanol, from haloalkanes like dichloromethane, from ethers like tetrahydrofuran or dioxane or from aromatic solvents like toluene or mixtures thereof. Also suited are mixtures of aliphatic alcohols such as methanol or ethanol with water or with dioxane. The preferred solvent is methanol or ethanol, even more preferred ethanol.

Suitable bases are inorganic bases selected from alkali or earth alkali- carbonates or - hydrogen carbonates or phosphates or hydrogenphosphates or dihydrogenphosphates or acetates or formates or organic bases selected from amines, alkali alcoholates or amidines. Organic bases are usually preferred. Typical representatives of organic bases are potassium tert-butylate or 1,8-Diazabicyclo[5.4.0]undec-7-ene (DBU), 1,4-Diazabicyclo(2.2.2)octane (DABCO) and 7-Methyl-1,5,7-triazabicyclo[4.4.0]dec-5-ene (MTBD), most preferred is DBU.

A substrate to catalyst ratio can expediently be chosen in a range of 100 to 10'000, preferably in a range of 1000 to 5000.

The chiral triol of formula I can be separated from the reaction mixture by evaporation of the solvent. Subsequent crystallization in a suitable solvent, typically in ketones like methyl isobutyl ketone or esters like isopropyl acetate renders the chiral triol of formula I in good yields, high purity and high enantiomeric excess.

### b) The asymmetric hydrogenation of the ketone of formula IIa in the presence of an in situ formed Ir-SpiroPAP catalyst.

In another embodiment the iridium spiro-pyridylamidophosphine catalyst (Ir-SpiroPAP catalyst) of formula IIIa or IIIb may be prepared in situ in the course of the asymmetric hydrogenation reaction by bringing together a suitable Iridium-pre catalyst complex with a spiro-pyridylamidophosphine ligand of the formula 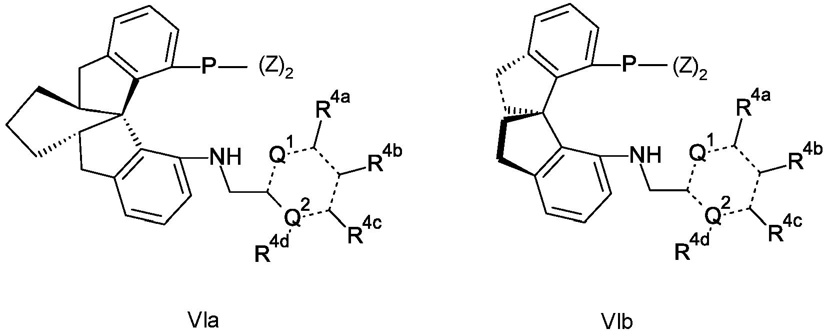 wherein
R^{4a}, R^{4b}, R^{4c} and R^{4d}, Q¹ and Q² and Z have the meanings as outlined above. Suitable Iridium-pre catalyst complex compounds are commercially available e.g. from Sigma Aldrich and can be selected e.g. from [Ir(cod)₂]BF₄, [IrCl(COD)]₂, [Ir(acac)(COD)], [Ir(OMe)(COD)]₂, [Ir(cod)₂]BARF, [Ir(cod)₂]PF6, wherein cod or COD has the meaning of cyclooctadiene, acac the meaning of acetylacetonate, BARF the meaning of tetrakis(3,5-bis(trifluoromethyl)phenyl)borate and OMe the meaning of methoxy.

Preferred Iridium-pre catalyst complex compound is [IrCl(COD)]₂.

Usually the iridium-pre catalyst complex compound and the spiro-pyridylamidophosphine ligand are typically mixed in the presence of the organic solvent and the base mentioned under embodiment a).

The substrate to Iridium ratio as a rule is adjusted between 100 and 10000, preferably between 1000 and 5000. The substrate to ligand ratio as a rule is adjusted between 0.5 and 1.5, preferably between 0.9 and 1.1.

The asymmetric hydrogenation conditions and the isolation of the chiral triol of formula I can otherwise be chosen as for the process of embodiment a). Also the preferred embodiments outlined in embodiment a) apply likewise.

### c) The asymmetric hydrogenation of the ketone of formula IIa to the ketone of formula IIb in the sole presence of an Ir-PEN catalyst and the subsequent asymmetric hydrogenation to the chiral triol of formula I in the presence of the Ir-SpiroPAP catalyst.

The iridium-phenylendiamine catalyst (Ir-PEN catalyst) are of the formula IVa or IVb, or enantiomers thereof 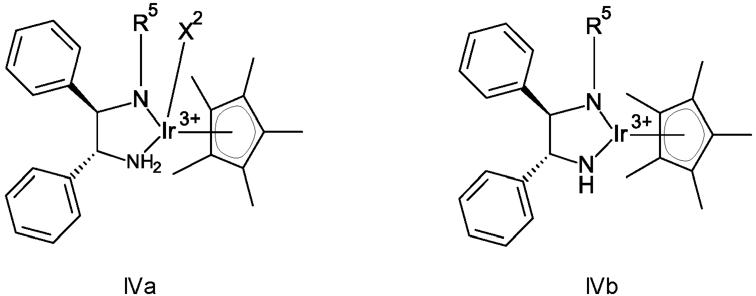 wherein,
R⁵ is C₁₋₆-alkylsulfonyl wherein the alkyl group is optionally substituted with one or more halogen atoms; with a 7,7-dimethyl-2-oxobicyclo[2.2.1] heptane-1-yl group or phenyl sulfonyl, wherein the phenyl group is optionally substituted by one or more C₁₋₆-alkyl groups and
X² is either a coordinated ligand or a counter anion selected from a C₁₋₆-alkylsulfonyloxy group which is optionally substituted with one or more halogen, atoms; from halogen, C₁₋₆-alkoxy, tetrahalogenoborate, hexahalogenoborate, tetrakis(3,5-bis(trihalogeno-C₁₋₆-alkyl)phenyl)borate, acetylacetonate, hexahalogenophosphine, p-tolylsulfonate (OTs) or trihalogenomethanesulfonate;
In a preferred embodiment the iridium-phenylendiamine catalyst (Ir-PEN catalyst) are of the formula IVa or IVb, or enantiomers thereof, wherein R⁵ is methylsulfonyl, trifluoromethylsulfonyl, 7,7-dimethyl-2-oxobicyclo[2.2.1] heptane-1-yl; tolylsulfonyl or 1,3,5-tri-i-propylphenyl sulfonyl;
X² is either a coordinated ligand or a counter anion selected from a methylsulfonyloxy group which is optionally substituted with one or more fluoro atoms; from halogen, methoxy, tetrafluoroborate (BF4), hexafluoroborate (BF6), tetrakis(3,5-bis(trifluoromethyl)phenyl)borate (barf), acetylacetonate (acac), hexafluorophosphate (PF6), p-tolylsulfonate (OTs) or trifluoromethanesulfonate (OTf;

In a further preferred embodiment the iridium-phenylendiamine catalyst (Ir-PEN catalyst) are of the formula IVa, or enantiomers thereof, wherein,
R⁵ is methylsulfonyl, trifluoromethylsulfonyl, 7,7-dimethyl-2-oxobicyclo[2.2.1] heptane-1-yl; tolylsulfonyl or 1,3,5-tri-i-propylphenyl sulfonyl;
X² is a trifluoromethylsulfonyl oxy group;

In a further preferred embodiment the iridium-phenylendiamine catalyst (Ir-PEN catalyst) are of the formula IVb, or enantiomers thereof, wherein,
R⁵ is methylsulfonyl, trifluoromethylsulfonyl, 7,7-dimethyl-2-oxobicyclo[2.2.1] heptane-1-yl; tolylsulfonyl or 1,3,5-tri-i-propylphenyl sulfonyl.

In a further preferred embodiment the iridium-phenylendiamine catalyst (Ir-PEN catalyst) are selected from compounds of the formula IVc and IVd

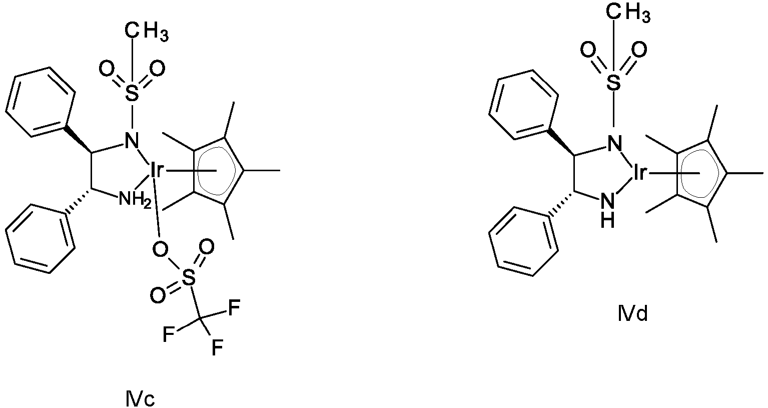

The asymmetric hydrogenation for the formation of the ketone of formula IIb of can be performed in the presence of suitable organic solvent at a hydrogen pressure of 5 bar to 100 bar, preferably of 30 bar to 70 bar and at a reaction temperature of 10 °C to 90°C, preferably of 20°C to 40°C.

The organic solvent can be selected from aliphatic alcohols selected from methanol, ethanol, isopropanol, tert-amylalcohol, from halogen substituted alcohols like trifluoroethanol, from haloalkanes like dichloromethane, from ethers like tetrahydrofuran or dioxane or from aromatic solvents like toluene or mixtures thereof. Also suited are mixtures of aliphatic alcohols such as methanol or ethanol with water or with dioxane. The preferred solvent is methanol or ethanol, even more preferred ethanol.

The reaction can be performed without the presence of a base.

However, bases are tolerated. Suitable bases are inorganic bases selected from alkali or earth alkali- carbonates or - hydrogen carbonates or phosphates or hydrogenphosphates or dihydrogenphosphates or acetates or formates or organic bases selected from amines, alkali alcoholates or amidines. Organic bases are usually preferred. Typical representatives of organic bases are potassium tert-butylate or 1,8-Diazabicyclo[5.4.0]undec-7-ene (DBU), 1,4-Diazabicyclo(2.2.2)octane (DABCO) and 7-Methyl-1,5,7-triazabicyclo[4.4.0]dec-5-ene (MTBD), most preferred is DBU.

A substrate to catalyst ratio can expediently be chosen in a range of 100 to 10000, preferably in a range of 500 to 1000.

The ketone of formula IIb can be separated from the reaction mixture by evaporation of the solvent. Subsequent crystallization in a suitable solvent, typically in an aliphatic alcohol like i-propanol renders the ketone of formula IIb in good yields, high purity and high enantiomeric excess. Alternatively the ketone of formula IIb is not isolated and is further hydrogenated to the chiral triol of formula I in the presence of the Ir-SpiroPAP catalyst.

The subsequent asymmetric hydrogenation can take place in the same manner as described in embodiment a)

### d) The asymmetric hydrogenation of the ketone of formula IIa in the presence of a mixture of an Ir-SpiroPAP catalyst and an Ir-PEN catalyst.

In this embodiment the asymmetric hydrogenation is performed in the presence of a mixture of the Ir-SpiroPAP catalyst and an Ir-PEN catalyst.

Typically the Ir-PEN catalyst catalyzes the first step of the reaction i.e. the transformation to the ketone of formula IIb faster and with a higher chiral selectivity than the Ir-SpiroPAP catalyst.

Therefore, regarding catalyst concentration of the two catalysts a higher Ir-PEN catalyst concentration is as a rule applied.

The substrate to Ir-PEN catalyst ratio can therefore expediently be chosen in a range of 100 to 10000 preferably in a range of 500 to 1000.

The substrate to Ir-Spiro-PAP catalyst ratio can expediently be chosen in a range of 100 to 10000, preferably in a range of 2500 to 7500.

The asymmetric hydrogenation can be performed in the presence of suitable organic solvent and a base at a hydrogen pressure of 5 bar to 100 bar, preferably of 30 bar to 70 bar and at a reaction temperature of 10°C to 90°C, preferably of 20°C to 40°C.

The organic solvent can be selected from aliphatic alcohols selected from methanol, ethanol, isopropanol, tert-amylalcohol, from halogen substituted alcohols like trifluoroethanol, from haloalkanes like dichloromethane, from ethers like tetrahydrofuran or dioxane or from aromatic solvents like toluene or mixtures thereof. Also suited are mixtures of aliphatic alcohols such as methanol or ethanol with water or with dioxane. The preferred solvent is methanol or ethanol, even more preferred ethanol.

Suitable bases are inorganic bases selected from alkali or earth alkali- carbonates or - hydrogen carbonates or phosphates or hydrogenphosphates or dihydrogenphosphates or acetates or formates or organic bases selected from amines, alkali alcoholates or amidines. Organic bases are usually preferred. Typical representatives of organic bases are potassium tert-butylate or 1,8-Diazabicyclo[5.4.0]undec-7-ene (DBU), 1,4-Diazabicyclo(2.2.2)octane (DABCO) and 7-Methyl-1,5,7-triazabicyclo[4.4.0]dec-5-ene (MTBD), most preferred is DBU.

The chiral triol of formula I can be separated from the reaction mixture by evaporation of the solvent. Subsequent crystallization in a suitable solvent, typically in ketones like methyl isobutyl ketone or esters like isopropyl acetate renders the chiral triol of formula I in good yields, high purity and high enantiomeric excess.

### e) The asymmetric hydrogenation of intermediate IIb, IIc or IId. in the sole presence of an Ir-SpiroPAP catalyst

The intermediates IIb, IIc or IId typically need not to be isolated and can directly be converted to the desired chiral triol of formula I.

Intermediate IIb can be prepared and isolated in accordance with embodiment c).

Also intermediate IIc or IId can in principle be isolated by interrupting the hydrogenation at the appropriate stage and individually be subjected to the asymmetric hydrogenation with either the Ir-Spiro PAP catalyst alone or in the presence of a mixture of an Ir-SpiroPAP catalyst and an Ir-PEN catalyst. The reaction conditions as described in the previous embodiments can likewise be applied.

As outlined above the embodiments a) to d) are preferred, more preferred are the embodiments a), b) and d) and embodiment d) is most preferred.

### Examples

### Abbreviations:

| | |
|---|---|
| EtOH | Ethanol |
| iPr₂O | Diisopropyl ether |
| MeOH | Methanol |
| DCM | Dichloromethane |
| Dioxane | 1,4-Dioxane |
| iPrOH | 2-Propanol |
| iPrOAc | Isopropyl acetate |
| tAmOH | tert-Amylalcohol |
| TFE | Trifluoroethanol |
| THF | Tetrahydrofuran |
| DBU | Diazabicycloundecene |
| DABCO | 1,4-Diazabicyclo(2.2.2)octane |
| MTBD | Triazabicyclodecene |
| DBN | 1,5-Diazabicyclo(4.3.0)non-5-ene |
| BIPY | Biypridine |
| COD | Cyclooctadiene |
| rt | Room temperature |
| IPC | In process control |
| T | Temperature |
| P | Hydrogen pressure |
| eq | Equivalent |
| rct | Reaction time |
| con | Conversion |
| exp | Experiment |
| S/C | Substrate-to-Catalyst ratio |
| S/L | Substrate-to-Ligand ratio |
| S/B | Substrate-to-Base ratio |
| S/Ir | Substrate-to-Iridium ratio |

| | |
|---|---|
| **1** | 4-(4-Chlorophenyl)-2-hydroxy-4-keto-butyric-2-en-acid ethyl ester (Note: 1H-NMR spectra data of **1** in D6-EtOH or CD₂Cl₂ confirmed the structure to be assigned as: 4-(4-chlorophenyl)-2-hydroxy-4-keto-butyric-2-en-acid ethyl ester. No hint was found for the presence of 4-(4-Chlorophenyl)-2-diketo-butyric acid ethyl ester) |
| *(R)***-3** = *(2R)***-3** | (2*R*)-4-(4-Chlorophenyl)-2-hydroxy-4-keto-butyric acid ethyl ester |
| *(R,R)-***4** *= trans-(2R,4R)*-**4** | (2*R*,4*R*)-4-(4-Chlorophenyl)-2,4-dihydroxy-butyric acid ethyl ester |
| *trans-***4** | mix of (*R,R*)*-***4** and (*S,S*)-**4** |
| *cis-***4** | mix of (*R,S*)*-***4** and *(S,R)-***4** |
| *(R,R)-5 = cis-(3R,5R)-5* | (3R,5R)-5-(4-Chlorophenyl)-3 -hydroxy-butyrolactone |
| *cis-5* | mix of *(R,R)-5* and (*S,S*)-*5* |
| *trans-5* | mix of (*R,S*)*-5* and (*S,R*)*-5* |
| *(R,R)-6* = *trans-(2R,4R)-6* | (2R,4R)-4-(4-Chlorophenyl)-butane-1,2,4-triol |
| *trans-6* | mix of *(R,R)-6* and (S,S)-6 |
| *cis-6* | mix of *(R,S)-6* and (S,R)-6 |
| 7 | 4-(Phenyl)-2-hydroxy-4-keto-butyric-2-en-acid ethyl ester |
| | (Note: 1H-NMR spectra data of 7 in CD₂Cl₂ confirmed the structure to be assigned as: 4-(Phenyl)-2-hydroxy-4-keto-butyric-2-en-acid ethyl ester. No hint was found for the presence of 4-(Phenyl)-2-diketo-butyric acid ethyl ester) |
| *(R,R)-8* = *trans-(2R,4R)-8* | (2R,4R)-4-(Phenyl)-butane-1,2,4-triol |
| *trans-8* | mix of (R,R)-8 and (S,S)-8 |
| *cis-8* | mix of (R,S)-8 and (S,R)-8 |

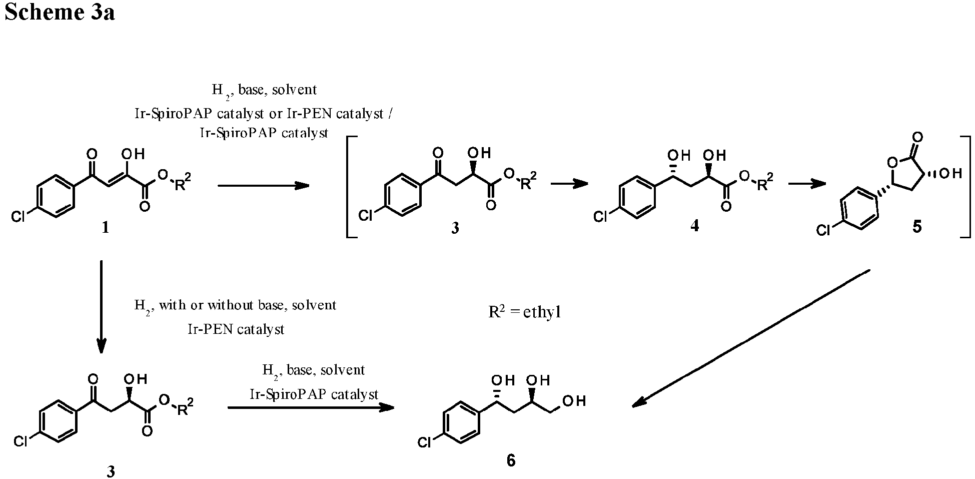

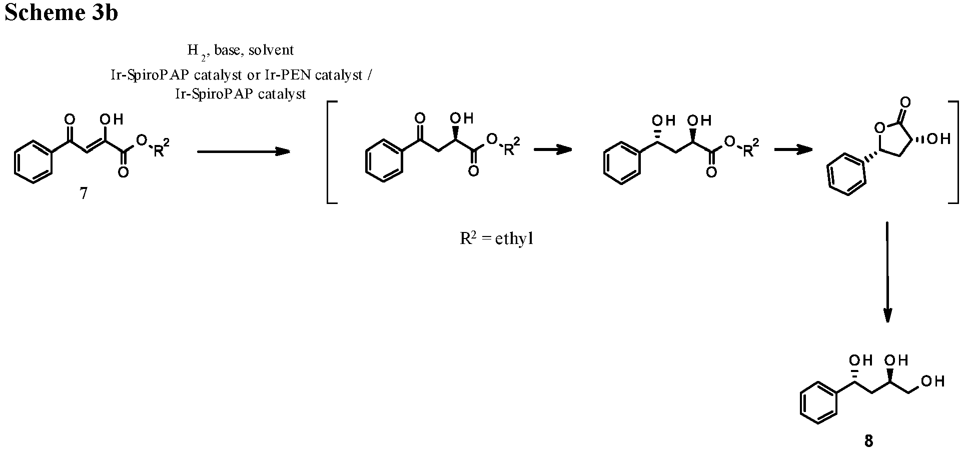

### Pre-catalysts, Catalyst and Ligands:

627-630 and 6051-6056 were prepared according to T. Ohjuma et al. Organic Letters, 2007, 9, 2565. All other (pre-) catalysts and ligands were commercially available e.g. from Strem, Sigma Aldrich, Jiuzhou Pharma.

| (Pre-) Catalysts and Ligands | | |
|---|---|---|
| Number | Abbreviation | Structure |
| **627** | [Ir(cp*)((S,S)-Ms-DPEN-2H)] CAS No 937378-51-3 | 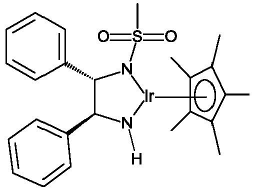 |
| **628** | [Ir(cp*)((S,S)-Ms-DPEN-H)(OTf)] CAS No 917756-11-7 | 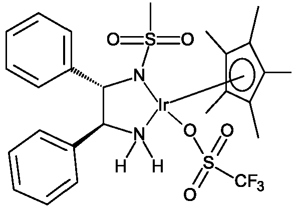 |
| **629** | [Ir(cp*)((R,R)-Ms-DPEN-2H)] CAS No 1263000-75-4 | 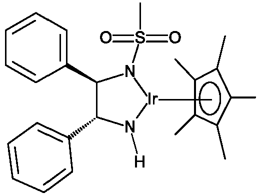 |
| **630** | [Ir(cp*)((R,R)-Ms-DPEN-H))(OTf)] CAS No 1201686-18-1 | 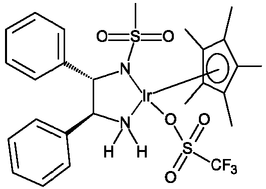 |
| **6051** | [Ir(cp*)((*R*,*R*)-Ts-DPEN-2H)] CAS No 401479-02-5 | 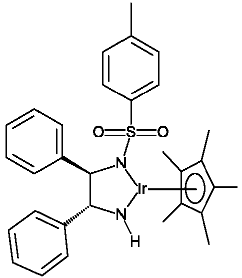 |
| **6052** | [Ir(cp*)((R,R,R)-Cs-DPEN-2H)] CAS No 895579-52-9 | 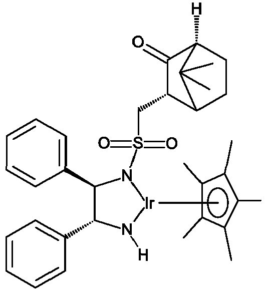 |
| **6053** | [Ir(cp*)((*S*,*S*)-TFMs-DPEN-2H)] CAS No 1807637-08-6 | 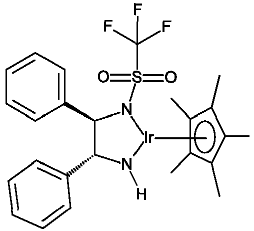 |
| **6054** | [Ir(cp*)((S,S)-TIPBs-DPEN-2H)] CAS No.1073339-77-1 | 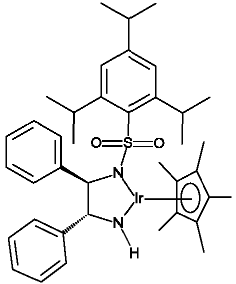 |
| **6055** | [Ir(cp*)((*S*,*S*)-Ts-1,3,5-MeDPEN-2H)] CAS No 2376389-13-6 | 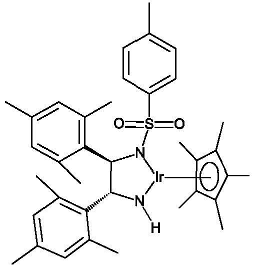 |
| **6056** | [Ir(cp*)((R,R)-Ts-DACH-2H)] CAS No. 1099830-96-2 | 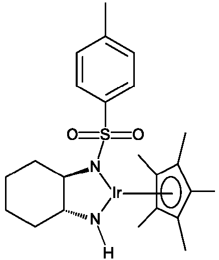 |
| **680** | [IrClH₂((*S*)-DTB-SpiroPAP-3-Me)] CAS No 1418483-59-6 | 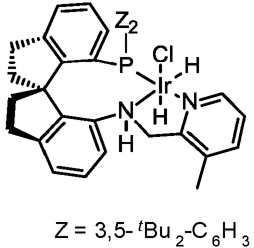 |
| | Available from Jiuzhou Pharma, CN Catalogue No. JZ-S033-2 | |
| **682** | [IrClH₂((*S*)-DTB-SpiroSAP)] CAS No not available | 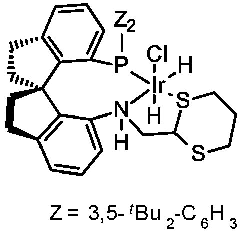 |
| | Available from Jiuzhou Pharma, CN Catalogue No. JZ-S034-2 | |
| **6046** | [IrClH₂((*S*,*S*,*S*)-DTB-PSpiroPAP-3-Me)] CAS No not available | 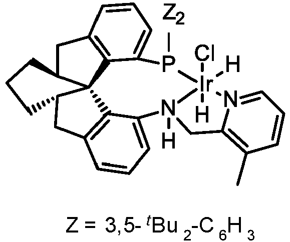 |
| | Available from Jiuzhou Pharma, CN Catalogue No. JZ-S036-1 | |
| **6048** | [IrClH₂((*S*)-DTB-SpiroPAP)] CAS No not available | 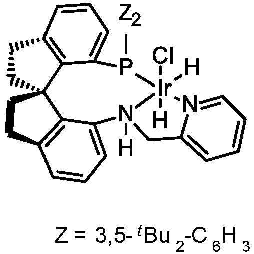 |
| | Available from Jiuzhou Pharma, CN Catalogue No. not available | |
| **6049** | [IrClH₂(*R*)-DTB-SpiroPAP-4-tBu)] CAS No not available | 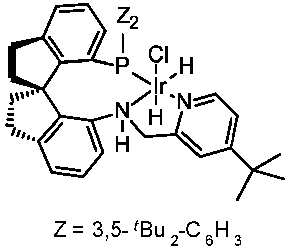 |
| | Available from Jiuzhou Pharma, CN Catalogue No. not available | |
| **6050** | [IrClH₂((*R*)-DTB-SpiroPAP-6-Me)] CAS No not available | 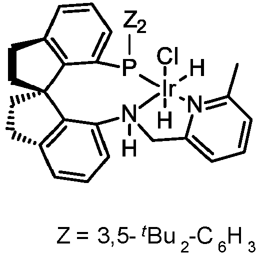 |
| | Available from Jiuzhou Pharma, CN Catalogue No. not available | |
| **1508** | (*S*)-DTB-SpiroPAP-3-Me CAS No not available | 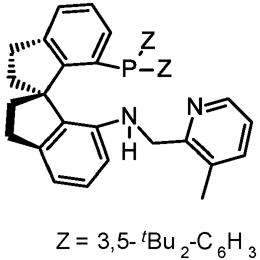 |
| | Available from Jiuzhou Pharma, CN Catalogue No. JZ-S022-2 | |
| **600** | [Ir(cod)₂]BF₄ CAS No 35138-23-9 | 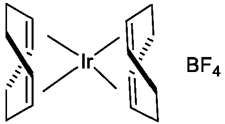 |
| **601** | [IrCl(COD)]₂ CAS No 12112-67-3 | 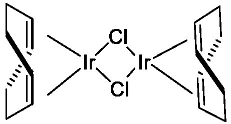 |
| **650** | [Ir(acac)(COD)] CAS No 12154-84-6 | 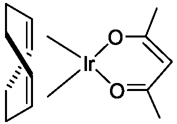 |
| **657** | [Ir(OMe)(COD)]₂ CAS No 12148-71-9 | 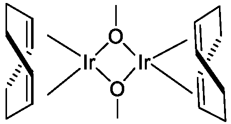 |

### Analytical Methods

### a) Achiral LC Method to determine the conversion and purities of 1, 3 and the cis- and trans-isomers of 4-6

| | |
|---|---|
| Stationary phase | Kinetex^{®} (2.6 µm PFP 100 Å, LC Column 50 x 4.6 mm) |
| Eluent: | A) Acetonitrile B) H2O + 5% Acetonitrile D) TBAHS Puffer (1g TBAHS in 800 mL Acetonitrile und 200 mL H2O). Pump program (gradient): 10 A : 80 B : 10 D → 80 A: 10 B: 10 D |
| Run time: | 16 min |
| Flow: | 1mL/min |
| Column oven temperature | 40°C |
| Injection volume: | 5 uL |
| Detection: | DAD 210 nm |
| Retention Times: | **1,** 13.23 min ; 3, 6.91 min ; *trans-4,* 6.04 min ; *cis-4,* 5.73 min ; *trans-5,* 5.49 min ; *cis-5,* 5.19 min ; *trans-6* 2.40 min ; *cis-6* 2.18 min |

### b) Chiral LC Method to determine the enantiomeric purity of 3

| | |
|---|---|
| Stationary phase: | Daicel Chiralpak IC-3, L = 150 mm, ID = 4.6 mm, 3.0 µm |
| Eluent: | A) H2O + 5% Acetonitrile B) Acetonitrile C) 6.25-6.35 g ammonium formate in 950.0 mL Water adjusted to pH 9.0 with ammonium hydroxide solution (25%) + 50.0 mL acetonitrile pump program (isocratic): 60 A : 30 B : 10 C |
| Run time: | 20 min |
| Flow: | 1mL/min |
| Column oven temperature: | 30°C |
| Injection volume: | 2.5 uL |
| Detection: | DAD 254 nm |
| Retention Times: | (*S*)-**3**, 10.60 min; (R)-**3**, 12.20 min |

### c) Chiral LC method to determine the enantiomeric purities of 3, 4, 5 and 6

| | |
|---|---|
| Stationary phase: | Daicel Chiralpak IB-N; L = 150mm, ID = 4.6mm, 3.0µm |
| Eluent: | A) CO2 B) Isopropanol, pump program (isocratic): 90 A : 10 B |
| Run time: | 9 min |
| Flow: | 3mL/min |
| Column oven temperature: | 20°C |
| Injection volume: | 5 uL |
| Detection: | DAD 220 nm |
| Retention Times: | **1,** 1.19 min; (R)-**3**, 1.85 min; (S)-**3**, 1.95 min; (R,R)-**4**, 2.24 min; (S,S)-**4**, 2.58 min; (*R,S*)-**4,** 3.09 min; (S,R)-**4**, |

| | |
|---|---|
| | 3.93 min; (*R,R*)*-***5*****,*** 3.08 min; (S,S)-**5**, 3.92 min; (*R,S*)-**5**, 2.33 min; (*S,R*)-**5,** 2.33 min; (R,R)-**6**, 5.11 min; (S,S)-**6**, 5.78 min; (*R,S*)-**6**, 6.37 min; (S,R)-**6**, 6.95 min |

### 1. Preparation of (R)-3 via asymmetric hydrogenation of 1

### Example 1.1

In a glove box under argon atmosphere, a 380 mL autoclave was charged with 1 (20.0 g, 78.5 mmol), 630 (60.2 mg, 78.3 x 10⁻⁶ mol, S/C 1'000) and EtOH (200 mL). The autoclave was sealed and removed from the glove box, connected to a hydrogen line, pressurized with hydrogen gas to 70 bar and heated to 30°C. Under stirring, the hydrogenation was ran at a constant hydrogen pressure of 70 bar. Reaction samples were taken after 1 h (50% conversion) and 2 h (>99.9 % conversion) to follow the progress of the reaction. After a total reaction time of 2.5 h, the autoclave was vented and allowed to cool to room temperature. The reaction mixture was transferred with aid of EtOH (20 mL) from the autoclave into a 500 mL round bottomed flask and the orange reaction solution rotatory evaporated at 40°C / 10 mbar to constant weight to yield crude (R)-3 (19.9 g) with 96.7 area-% purity and 94.3% ee. 0.9% of *trans-4* was detected as major impurity (note: *trans-4* demonstrated to have limited stability and converted during handling and storage gradually into *trans-*5)*.*

Next, crude (R)-3 (5.00 g) was dissolved in iPr₂O (25 mL) at 60°C. The clear solution was allowed to cool to 0°C within 6 h and stirred at this temperature for another 1.5 h. The formed white crystals were filtered, washed with 9 mL of ice cold iPr₂O and dried for 1 h at 40°C under vacuum (10 mbar) to afforded pure (R)-3 (4.15 g, 82% yield) with 99.9 area-% purity and 99.6% ee.

### Analytical data for 3

LC-MS ESI (m/z): 256.0 [M+]
¹H-NMR (CDCl₃, 600 MHz): δ ppm 7.89 (d, J=8.8 Hz, 2H), 7.41 - 7.50 (m, 3H), 4.65 (td, J=5.8, 3.8 Hz, 1H), 4.28 (q, J=7.2 Hz, 2H), 3.46 - 3.53 (m, 1H), 3.38 - 3.45 (m, 1H), 3.27 (d, J=5.6 Hz, 1H), 1.29 (t, J=7.1 Hz, 3H)

### Analytical data for trans-4

GC-MS ESI (m/z): 258.0 [M+]
1H NMR (DMSO-D6, 600 MHz): δ ppm 7.35 - 7.38 (m, 2 H), 7.32 - 7.35 (m, 2 H), 5.44 (br s, 2 H), 4.73 (br d, J=9.6 Hz, 1 H), 4.25 (br d, J=8.6 Hz, 1 H), 4.06 (q, J=7.1 Hz, 2 H), 1.53 - 1.78 (m, 1 H), 1.45 - 1.99 (m, 1 H), 1.17 (t, J=7.1 Hz, 3 H)

### Example 1.2:

In a glove box under argon atmosphere, a 380 mL autoclave was charged with 1 (20.0 g, 78.5 mmol), 629 (48.4 mg, 78.3 x 10⁻⁶ mol, S/C 1'000) and EtOH (200 mL). The autoclave was sealed and removed from the glove box, connected to a hydrogen line, pressurized with hydrogen gas to 70 bar and heated to 30°C. Under stirring, the hydrogenation was ran at a constant hydrogen pressure of 70 bar. A reaction sample was taken after 3.5 h (98% conversion) to follow the progress of the reaction. After a total reaction time of 4 h, the autoclave was vented and allowed to cool to room temperature. The reaction mixture was transferred with aid of EtOH (20 mL) from the autoclave into a 500 mL round bottomed flask and the orange reaction solution rotatory evaporated at 40°C / 10 mbar to constant weight to yield crude (R)-3 (20.0 g) with 94 area-% purity and 94.5% ee. 1.3% of *trans-4* was detected as major impurity. Next, crude (R)-3 (20.0 g) was dissolved in iPr₂O (200 mL) at 40°C. The clear solution was then allowed to cool to 0°C within 6 h and stirred at this temperature for another 1.5 h. The formed white crystals were filtered, washed with 45 mL of ice cold iPr₂O and dried for 1 h at 40°C under vacuum (10 mbar) to afforded 15.62 g of pure (R)-3 (15.62 g, 78% yield) with 99.2 area-% purity and 99.8% ee.

### Examples 1.3 - 1.6

In analogy to *Example 1.1,* 1 (0.5 g, 1.96 mmol) was hydrogenated for 20 h in EtOH (5 mL) and the presence of the catalysts as listed in *Table 1* at 30°C and an initial hydrogen pressure of 70 bar H₂.

**Table 1:**

| exp | catalyst | conv | **3** | *(R)-**3*** | *trans-**4*** |
|---|---|---|---|---|---|
| | | [%] | [area-%] | [% ee] | [area-%] |
| 1.3 | **629** | >99.9 | 90 | 95.0 | 7 |
| 1.4 | **6051** | >99.9 | 95.8 | 95.0 | 2.2 |
| 1.5 | **6052** | >99.9 | 94 | 92 | 1.9 |
| 1.6 | **6053** | 60 | 40 | 63 (S) | 0 |

### Examples 1.7 - 1.10

In analogy to *Example 1.1,* 1 (0.25 g, 0.98 mmol) was hydrogenated for 2 h in EtOH (5 mL) and the presence of the catalysts as listed in *Table 2* at 30°C and an initial hydrogen pressure of 70 bar H₂.

**Table 2:**

| exp | catalyst | conv | **3** | (*R*)-**3** | *trans*-**4** |
|---|---|---|---|---|---|
| | | [%] | [area-%] | [% ee] | [area-%] |
| 1.7 | **629** | 59 | 52 | 95.0 | 0 |
| 1.8 | **6054** | 21 | 9 | n.d. | 0 |
| 1.9 | **6055** | 9 | 7 | n.d. | 0 |
| 1.10 | **6056** | 42 | 38 | 15 | 0 |

### Examples 1.11 - 1.14

In analogy to *Example 1.1,* 1 (0.25 g, 0.98 mmol) was hydrogenated for 2 h in EtOH (5 mL) at 30°C in the presence of the catalysts (S/C 1'000) and initial hydrogen pressures as listed in *Table 3.*

**Table 3:**

| exp | catalyst | p | conv | **3** | (*R*)-**3** | *trans-**4*** |
|---|---|---|---|---|---|---|
| | | [bar] | [%] | [area-%] | [% ee] | [area-%] |
| 1.11 | **629** | 30 | 75 | 71 | n.d. | 0 |
| 1.12 | **629** | 50 | 89 | 87 | n.d. | 0 |
| 1.13 | **630** | 30 | >99.9 | 96.5 | 95.7 | 0 |
| 1.14 | **630** | 50 | >99.9 | 95.9 | 95.7 | 0.4 |

### Examples 1.15 - 1.20

In analogy to *Example 1.1,* 1 (0.25 g, 0.98 mmol) was hydrogenated for 2 h in EtOH (5 mL) at 30°C and an initial hydrogen pressures of 70 bar in the presence of various amounts of catalysts and DBU as base as listed in *Table 4.*

**Table 4:**

| exp | catalyst | S/C | DBU | conv | **3** | *(R)-**3*** | *trans-**4*** |
|---|---|---|---|---|---|---|---|
| | | | [eq] | [%] | [area-%] | [% ee] | [area-%] |
| 1.15 | **630** | 1'000 | - | >99.9 | 96.3 | 95.6 | 0.3 |
| 1.16 | **630** | 1'000 | 10 | 95.0 | 94.5 | 96.5 | 0 |
| 1.17 | **630** | 500 | 50 | >99.9 | 97 | n.d. | 1.4 |
| 1.18 | **630** | 1000 | 50 | 98.3 | 96.9 | 96.4 | 0 |
| 1.19 | **630** | 2'000 | 50 | 56 | 55 | n.d. | 0 |
| 1.20 | **630** | 2'000 | - | 46 | 46 | n.d. | 0 |

### 2. Preparation of (R,R)-6 via asymmetric hydrogenation of 1

### Examples 2.1

In a glove box under argon atmosphere, a 380 mL autoclave was charged with 1 (10.0 g, 39.3 mmol), 680 (38.4 mg, 39.3 x 10⁻⁶ mol, S/C 1'000), DBU (597.8 mg, 3.93 mmol, S/B 10) and EtOH (200 mL). The autoclave was sealed and removed from the glove box, connected to a hydrogen line and pressurized with hydrogen gas to 70 bar and heated to 30°C. Under stirring, the hydrogenation was run at a constant hydrogen pressure of 70 bar. After a total reaction time of 20 h (>99.9% conversion), the autoclave was vented and allowed to cool to room temperature. The reaction mixture was transferred with aid of EtOH (20 mL) from the autoclave into a 500 mL round bottomed flask and the orange reaction solution rotatory evaporated at 40°C / 10 mbar to constant weight to yield crude 6 (9.0 g) with 98.4 area-% purity (DBU not integrated) and a *trans*/*cis* ratio of 7.7. (R,R)-6 was obtained with 98.8% ee.

### Analytical data for cis-5

GC-MS ESI (m/z): 212.0 [M+]
¹H-NMR (DMSO-D6, 600 MHz): δ 7.47 - 7.51 (m, 2H), 7.42 (d, J=8.3 Hz, 2H), 6.02 (br s, 1H), 5.40 (dd, J=10.8, 5.4 Hz, 1H), 4.62 (dd, J=10.7, 8.6 Hz, 1H), 2.89 (ddd, J= 12.2, 8.2, 5.4 Hz, 1H), 1.93 (dt, J=12.1, 11.0 Hz, 1H)

### Analytical data for trans-5

GC-MS ESI (m/z): 212.0 [M+]
1H-NMR (DMSO-D6, 600 MHz): δ 7.47 (d, J=8.7 Hz, 2 H), 7.40 - 7.42 (m, 2 H), 6.18 (br d, J=5.2 Hz, 1 H), 5.68 (t, J=6.7 Hz, 1H), 4.38 (dt, J=7.0, 4.9 Hz, 1 H), 2.44 - 2.48 (m, 1 H), 2.36 - 2.42 (m, 1 H)

### Analytical data for trans-6

GC-MS ESI (m/z): 216.0 [M+]
¹H NMR (400 MHz, DMSO) δ 7.40 - 7.31 (m, 4H), 5.23 (d, *J =* 4.9 Hz, 1H), 4.75 (dd, *J =* 9.9, 4.8 Hz, 1H), 4.50 (dd, *J =* 6.5, 5.5 Hz, 2H), 3.68 - 3.67 (m, 1H), 3.30 - 3.24 (m, 2H), 1.67 - 1.61 (m, 1H), 1.44 - 1.39 (m, 1H).
¹³C NMR (101 MHz, DMSO) δ 146.6, 131.3, 128.4, 127.9, 68.7, 68.6, 66.8, 44.3.

### Analytical data for cis-6

GC-MS ESI (m/z): 216.0 [M+]
¹H-NMR (CDCl₃, 600 MHz): δ ppm 7.31-7.34 (m, 2H), 7.32 (s, 2H), 4.98 (dd, J=9.9, 2.5 Hz, 1H), 4.04 (br d, J= 2.4 Hz, 1H), 3.45-3.70 (m, 2H), 2.76 (s, 1H), 1.65-1.95 (m, 2H), 1.08 (s, 2H).
¹³C_NMR (CDCl₃, 151 MHz) δ 142.7, 133.3, 128.7, 127.1, 73.82, 72.3, 66.7, 41.6.

### Examples 2.2

In a glove box under argon atmosphere, a 380 mL autoclave was charged with 1 (10.0 g, 39.3 mmol), 601 (29.4 mg, 19.6 x 10⁻⁶ mol, S/Ir 1'000), **1508** (13.2 mg, 39.3 x 10⁻⁶ mol, S/L 1'000), DBU (597.8 mg, 3.93 mmol, S/B 10) and EtOH (200 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar. After a total reaction time of 20 h (>99.9% conversion), the autoclave was vented and allowed to cool to room temperature. The reaction mixture was transferred with aid of EtOH (20 mL) from the autoclave into a 500 mL round bottomed flask and the orange reaction solution rotatory evaporated at 40°C / 10 mbar to constant weight to yield crude 6 (9.1 g) with 96.5 area-% purity (DBU not integrated) and a *trans*/*cis* ratio of 8.3. (R,R)-6 was obtained with >99.9% ee.

### Specified impurity: trans-5 (0.8%)

### Examples 2.3

In a glove box under argon atmosphere, a 380 mL autoclave was charged with 1 (10.0 g, 39.3 mmol), 601, 29.4 mg, 19.6 x 10⁻⁶ mol, S/Ir 1'000), **1508** (13.2 mg, 39.3 x 10⁻⁶ mol, S/L 1'000), KOtBu (437.6 mg, 3.93 mmol, S/B 10) and EtOH (200 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar. After a total reaction time of 42 h (>99.9% conversion), the autoclave was vented and allowed to cool to room temperature. The reaction mixture was transferred with aid of EtOH (20 mL) from the autoclave into a 500 mL round bottomed flask and the orange reaction solution rotatory evaporated at 40°C / 10 mbar to constant weight to yield crude 6 (9.0 g) with 84.2 area-% purity and a *trans*/*cis* ratio of 8.0. (R,R)-6 was obtained with 98.6% ee.

### Specified impurity: trans-5 (0.9%)

### Examples 2.4 - 2.8

In analogy to *Example 2.1,* 1 (0.25 g, 0.98 mmol) was hydrogenated for 20 h in EtOH (5 mL) at 30°C and an initial hydrogen pressure of 70 bar in the presence KOtBu (4.9 x 10⁻⁵ mol, S/B 20) and of the catalysts (S/C 1'000) as listed in *Table 5.*

**Table 5:**

| exp | cat | conv | **3** | *trans-**4*** | *trans-**5*** | **6** | *(R,R)-**6*** | **6** |
|---|---|---|---|---|---|---|---|---|
| | | [%] | [area-%] | [area-%] | [area-%] | [area-%] | [%ee] | [*trans*/*cis*] |
| 2.4 | **682** | >99.9 | 0 | 51 | 27 | 15 | 99.9 | 35 |
| 2.5 | **6046** | 85 | 67 | 0 | 0 | 0 | - | - |
| 2.6 | **6048** | >99.9 | 0.3 | 0 | 1.2 | 93 | 99.9 | 7.8 |
| 2.7 | **6049** | 99.1 | 1.1 | 0 | 1.4 | 88 | 99.8 | 7.2 |
| 2.8 | **6050** | 77 | 56 | 0.4 | 0 | 0 | - | - |

### Examples 2.9 - 2.16

In analogy to *Example 2.2,* 1 (0.25 g, 0.98 mmol) was hydrogenated for 20 h in EtOH (5 mL) at 30°C and an initial hydrogen pressure of 70 bar in the presence of 1508 (0.98 x 10⁻⁶ mol, S/L 1'000), the presence or absence of DBU (0.98 x 10⁻⁴ mol, S/B 10) and the presence of a pre-catalyst (S/Ir 1'000) as listed in *Table 6.*

**Table 6:**

| exp | pre-catalyst | base | conv | **3** | trans-**4** | trans-**5** | **6** | *(R,R)*-**6** | **6** |
|---|---|---|---|---|---|---|---|---|---|
| | | | [%] | [area-%] | [area-%] | [area-%] | [area-%] | [%ee] | [*trans*/*cis*] |
| 2.9 | **600** | DBU | 99.5 | 0 | 1.0 | 1.5 | 93 | 99.9 | 8.3 |
| 2.10 | **601** | DBU | 99.6 | 0 | 0 | 0.7 | 96.7 | 99.6 | 7.5 |
| 2.11 | **650** | DBU | 99.7 | 0 | 0 | 0.8 | 96.0 | 99.6 | 7.3 |
| 2.12 | **657** | DBU | 99.3 | 0 | 0 | 0.7 | 97.3 | 99.5 | 6.9 |
| 2.13 | **600** | - | 82 | 69 | 1.0 | 0 | 0 | - | - |
| 2.14 | **601** | - | 95 | 52 | 7 | 1.7 | 0 | - | - |
| 2.15 | **650** | - | 75 | 64 | 0.6 | 0 | 0 | - | - |
| 2.16 | **657** | - | >99.9 | 20 | 70 | 0.5 | 0 | - | - |

### Examples 2.17 - 2.29

In analogy to *Example 2.1, 1* (0.10 g, 0.39 mmol or 0.25 g, 0.98 mmol) was hydrogenated for 20 h in EtOH (2 mL for 0.10 g scale experiments, resp. 4 mL for 0.25 g experiments) at 30°C and an initial hydrogen pressure of 70 bar in the presence of 680 (9.8 x 10⁻⁷ mol, S/C 1'000), the presence of a base (S/B 10) as listed in *Table 7.*

**Table 7:**

| exp | base | conv | 3 | *trans-4* | *trans-5* | 6 | (R,R)-6 | 6 |
|---|---|---|---|---|---|---|---|---|
| | | [%] | [area-% | [area-%] | [area-%] | [area-%] | [%ee] | *[translcis]* |
| 2.17 | - | 8 | 6 | 0 | 0 | 0 | - | - |
| 2.18 | KOtBu | >99.9 | 0 | 1.6 | 0 | 85 | 99.9 | 13 |
| 2.19 | NaHCO₃ | 79 | 67 | 2.4 | 0 | 0 | - | - |
| 2.20 | DBU | >99.9 | 0 | 3.5 | 0 | 91 | 99.9 | 20 |
| 2.21 | NEt₃ | >99.9 | 0 | 62 | 34 | 0.7 | - | - |
| 2.22 | KH₂PO₄ | 25 | 20 | 0 | 0 | 0 | - | - |
| 2.23 | Cs₂CO₃ | 96.7 | 0 | 0 | 0 | 85 | 99.6 | 12 |
| 2.24 | NaOCHO | 51 | 40 | 0 | 0 | 0 | - | - |
| 2.25 | NaOAc | 57 | 46 | 0 | 0 | 0 | - | - |
| 2.26 | DABCO | 93 | 86 | 3.4 | 0 | 0 | - | - |
| 2.27 | DBN | >99.9 | 0 | 26 | 50 | 17 | n.d. | 38 |
| 2.28 | BIPY | 6 | 11 | 0 | 0 | 0 | - | - |
| 2.29 | MTBD | >99.9 | 0 | 0 | 0.6 | 96.3 | 99.9 | 8.3 |

### Examples 2.30 - 2.42

In analogy to *Example 2.1,* 1 (0.25 g, 0.98 mmol) was hydrogenated for 20 h at 30°C and an initial hydrogen pressure of 70 bar in the presence of 680 (either 0.98 x 10⁻⁶ mol, S/C 1'000 or 0.20 x 10⁻⁶ mol, S/C 5'000), the presence of KOtBu (0.98 mmol, S/B 10) and a solvent or solvent mixtures (5 mL) as listed in *Table 8.*

**Table 8:**

| exp | solvent | S/C | conv | 3 | *trans-4* | *trans-5* | 6 | *(R,R)-*6 | 6 |
|---|---|---|---|---|---|---|---|---|---|
| | | | [%] | [area-%] | [area-%] | [area-%] | [area-%] | [%ee] | [*trans*/*cis*] |
| 2.30 | EtOH | 1'000 | >99.9 | 0 | 1.6 | 0 | 85 | 99.8 | 13 |
| 2.31 | MeOH | 1'000 | >99.9 | 0 | 0 | 0 | 92 | 97.0 | 4.0 |
| 2.32 | EtOH | 5'000 | 53 | 14 | 0 | 0 | 0 | - | - |
| 2.33 | MeOH | 5'000 | 68 | 20 | 5 | 0 | 0 | - | - |
| 2.34 | iPrOH | 5'000 | 23 | 5 | 0 | 0 | 0 | - | - |
| 2.35 | tAmOH | 5'000 | 15 | 2.0 | 0 | 0 | 0 | - | - |
| 2.36 | DCM | 5'000 | 22 | 6 | 0 | 0 | 0 | - | - |
| 2.37 | THF | 5'000 | 19 | 8 | 0 | 0 | 0 | - | - |
| 2.38 | toluene | 5'000 | 16 | 4.4 | 0 | 0 | 0 | - | - |
| 2.39 | TFE | 5'000 | 52 | 16 | 2.9 | 0 | 0 | - | - |
| 2.40 | dioxane | 5'000 | 3.8 | 0 | 0 | 0 | 0 | - | - |
| 2.41 | EtOH / H₂O 95:5 | 5'000 | 99.0 | 47 | 0 | 0 | 0 | - | - |
| 2.42 | EtOH / dioxane 1:1 | 5'000 | 28 | 6 | 0 | 0 | 0 | - | - |

### Examples 2.43 - 2.51

In analogy to *Example 2.1,* **1** (0.20 g, 0.79 mmol) was hydrogenated for 20 h in EtOH (5 mL) the presence of **680** (either 0.79 x 10⁻⁶ mol, S/C 1'000 or 0.16 x 10⁻⁶ mol S/C 5'000), the presence of various amounts of KOtBu, different temperatures and initial hydrogen pressures all as listed in *Table 9*.

**Table 9:**

| exp | S/C | S/B | T | p | conv | **3** | *trans* **-4** | *trans* **-5** | **6** | (*R,R*)**-6** | **6** |
|---|---|---|---|---|---|---|---|---|---|---|---|
| | | | | [bar] | [%] | [area -%] | [area-%] | [area-% ] | [area-% ] | [%ee] | *[trans* /*cis*] |
| 2.43 | 1000 | 1000 | 30 | 70 | 30 | 25 | 0 | 0 | 0 | - | - |
| 2.44 | 1000 | 100 | 30 | 70 | 75 | 65 | 0 | 0 | 0 | - | - |
| 2.45 | 1000 | 10 | 30 | 70 | >99.9 | 0 | 0 | 0 | 87 | 98.8 | 7.3 |
| 2.46 | 1000 | 1000 | 60 | 70 | 85 | 76 | 0 | 0 | 0 | - | - |
| 2.47 | 1000 | 100 | 60 | 70 | >99.9 | 0 | 0 | 0 | 88 | 97.9 | 7.3 |
| 2.48 | 1000 | 10 | 60 | 70 | >99.9 | 0 | 0 | 0 | 8 | 96.5 | 5.2 |
| 2.49 | 5000 | 1 | 30 | 70 | 17 | 0 | 1.2 | 0 | 13 | 99.9 | 7.8 |
| 2.50 | 5000 | 1 | 30 | 100 | 16 | 0 | 1.3 | 0 | 12 | 99.9 | 8.0 |
| 2.51 | 5000 | 1 | 90 | 100 | >99.9 | 0 | 4.6 | 0 | 0 | - | - |

### Example 2.52

In a glove box under argon atmosphere, a 180 mL autoclave was charged with **1** (1.00 g, 3.93 mmol), **629** (1.21 mg, 1.96 x 10⁻⁶ mol, S/C 2'000), **680** (1.92 mg, 1.96 x 10⁻⁶ mol, S/C 2'000), DBU (59.8 mg, 3.93 x 10⁻⁴ mol, S/B 10) and EtOH (20 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar. Reaction samples were taken at different time points (see *Table 10*) to follow the progress of the reaction.

**Table 10:**

| rct | conv | 3 | *trans***-4** | *trans***-5** | **6** | *(R,R)*-**6** | **6** |
|---|---|---|---|---|---|---|---|
| [h] | [%] | [area-%] | [area-%] | [area-%] | [area-%] | [%ee] | [*trans*/*cis*] |
| 1 | 16 | 11 | 0 | 0 | 0 | - | - |
| 2 | 51 | 46 | 0.1 | 0 | 0.2 | - | - |
| 4 | 96.4 | 76 | 11 | 7 | 0.3 | - | - |
| 6 | 95.9 | 0.7 | 30 | 41 | 17 | 99.9 | 48 |
| 8 | 97.5 | 0 | 2.0 | 14 | 74 | 99.9 | 55 |
| 24 | >99.9 | 0 | 0 | 0.8 | 98.4 | 99.9 | 14 |

### Example 2.53

In a glove box under argon atmosphere, a 180 mL autoclave was charged with **1** (1.00 g, 3.93 mmol), **629** (2.42 mg, 3.93 x 10⁻⁶ mol, S/C 1'000), **680** (0.77 mg, 0.79 x 10⁻⁶ mol, S/C 5'000), DBU (30.0 mg, 1.97 x 10⁻⁴ mol, S/B 20) and EtOH (20 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar. Reaction samples were taken at different time points (see *Table 11*) to follow the progress of the reaction.

**Table 11**

| rct | conv | **3** | *trans***-4** | *trans*-**5** | **6** | *(R,R)*-**6** | **6** |
|---|---|---|---|---|---|---|---|
| [h] | [%] | [area-%] | [area-%] | [area-%] | [area-%] | [%ee] | [*trans*/*cis*] |
| 1 | 52 | 50 | 0 | 0 | 0 | - | - |
| 2 | 84 | 82 | 0.3 | 0 | 0 | - | - |
| 4 | 99.1 | 76 | 16 | 6 | 0 | - | - |
| 6 | 98.5 | 23 | 46 | 24 | 1.2 | - | - |
| 8 | 97.7 | 3.8 | 49 | 28 | 13 | n.d. | n.d. |
| 22 | >99.9 | 0 | 0.9 | 2.7 | 94 | 99.9 | 32 |

### Example 2.54

In a glove box under argon atmosphere, a 180 mL autoclave was charged with **1** (1.00 g, 3.93 mmol), **629** (2.42 mg, 3.93 x 10⁻⁶ mol, S/C 1'000), **680** (0.77 mg, 0.79 x 10⁻⁶ mol, S/C 5'000), DBU (5.98 mg, 3.93 x 10⁻⁵ mol, S/B 100) and EtOH (20 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar. Reaction samples were taken at different time points (see *Table 12*) to follow the progress of the reaction.

**Table 12:**

| rct | conv | **3** | *trans*-**4** | *trans*-**5** | **6** | *(R,R)***-6** | **6** |
|---|---|---|---|---|---|---|---|
| [h] | [%] | [area-%] | [area-%] | [area-%] | [area-%] | [%ee] | [*trans*/*cis*] |
| 1 | 15 | 13 | 0 | 0 | 0 | - | - |
| 2 | 49 | 47 | 0 | 0 | 0 | - | - |
| 4 | 83 | 76 | 1.1 | 0 | 0 | - | - |
| 6 | 98.3 | 81 | 9 | 1.6 | 0.4 | - | - |
| 8 | 98.9 | 2.3 | 28 | 14 | 53 | 99.9 | 55 |
| 22 | >99.9 | 0 | 0 | 0 | 98.0 | 99.9 | 16 |

### Example 2.55

In a glove box under argon atmosphere, a 180 mL autoclave was charged with **1** (1.00 g, 3.93 mmol), **630** (3.00 mg, 3.93 x 10⁻⁶ mol, S/C 1'000) and EtOH (20 mL). The autoclave was sealed and removed from the glove box, connected to a hydrogen line and pressurized with hydrogen gas to 30 bar and heated to 30°C. Under stirring, the hydrogenation was ran at a constant hydrogen pressure of 30 bar for 4h. Afterward the pressure was released to 1-2 bar and the autoclave returned to the glove box where under argon atmosphere it was opened and charged with **680** (0.77 mg, 0.79 x 10⁻⁶ mol, S/C 5'000), DBU (30.0 mg, 1.97 x 10⁻⁴ mol, S/B 20). The autoclave was sealed again and removed from the glove box, connected to a hydrogen line and pressurized with hydrogen gas to 70 bar and heated to 30°C. Under stirring, the hydrogenation was ran at a constant hydrogen pressure of 70 bar. The reaction was continued for 18 h at to 70 bar and heated to 30°C. Reaction samples were taken at different time points (see *Table 13*) to follow the progress of the reaction.

**Table 13:**

| rct | conv | **3** | | *trans-***4** | *trans*-**5** | **6** | *(R,R)*-**6** | **6** |
|---|---|---|---|---|---|---|---|---|
| [h] | [%] | [area-%] | [%ee] | [area-%] | [area-%] | [area-%] | [%ee] | [*trans*/*cis*] |
| 1 | 54 | 52 | - | 0 | 0 | 0 | - | - |
| 2 | 87 | 85 | 95 | 0 | 0 | 0 | - | - |
| 4 | >99.9 | 93 | 95.2 | 0.4 | 0 | 0 | - | - |
| 6 | >99.9 | 31 | - | 19 | 18 | 0.7 | - | - |
| 23 | >99.9 | 0.8 | - | 23 | 10 | 56 | 99.9 | 78 |

### Example 2.56

In a glove box under argon atmosphere, a 180 mL autoclave was charged with **1** (1.00 g, 3.93 mmol), **630** (3.00 mg, 3.93 x 10⁻⁶ mol, S/C 1'000), **680** (0.77 mg, 0.79 x 10⁻⁶ mol, S/C 5'000), DBU (12.0 mg, 7.86 x 10⁻⁵ mmol, S/B 50) and EtOH (20 mL). The autoclave was sealed and removed from the glove box, connected to a hydrogen line and pressurized with hydrogen gas to 30 bar and heated to 30°C. Under stirring, the hydrogenation was ran at a constant hydrogen pressure of 30 bar for 4 h. Afterward the pressure was increased to 70 bar and the reaction carried out for additional 19 h. Reaction samples were taken at different time points (see *Table 14)* to follow the progress of the reaction.

**Table 14:**

| rct | conv | **3** | | *trans-***4** | *trans-***5** | **6** | *(R,R)-***6** | 6 |
|---|---|---|---|---|---|---|---|---|
| [h] | [%] | [area-%] | [%ee] | [area-%] | [area-%] | [area-%] | [%ee] | [*trans*/*cis*] |
| 1 | 34 | 34 | - | - | - | - | - | - |
| 2 | 48 | 48 | 92.4 | - | - | - | - | - |
| 4 | 67 | 66 | 91.9 | 0.2 | - | - | - | - |
| 6 | 74 | 73 | 91.1 | 0.3 | - | - | - | - |
| 23 | >99.9 | 0.9 | - | 3.8 | 0.3 | 92 | >99.9 | 20 |

### Example 2.57

In a glove box under argon atmosphere, a 180 mL autoclave was charged with **1** (1.00 g, 3.93 mmol), **630** (3.00 mg, 3.93 x 10⁻⁶ mol, S/C 1'000), **680** (0.77 mg, 0.79 x 10⁻⁶ mol, S/C 5'000), DBU (12.0 mg, 7.86 x 10⁻⁵ mmol, S/B 50) and EtOH (20 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar for 23 h. Reaction samples were taken at different time points (see *Table 15*) to follow the progress of the reaction.

**Table 15:**

| rct | conv | **3** | | *trans-***4** | *trans-***5** | **6** | *(R,R)-***6** | **6** |
|---|---|---|---|---|---|---|---|---|
| [h] | [%] | [area-%] | [%ee] | [area-%] | [area-%] | [area-%] | [%ee] | [*trans*/*cis*] |
| 1 | 30 | 30 | - | - | - | - | - | - |
| 2 | 62 | 61 | 92.6 | - | - | - | - | - |
| 4 | 96.0 | 91 | 92.7 | 3.9 | - | - | - | - |
| 6 | >99.9 | 43 | - | 40 | 16 | 0.4 | - | - |
| 23 | >99.9 | - | - | - | - | 98.1 | >99.9 | 15 |

### Example 2.58

In a glove box under argon atmosphere, a 180 mL autoclave was charged with **1** (1.00 g, 3.93 mmol), **630** (3.00 mg, 3.93 x 10⁻⁶ mol, S/C 1'000), **680** (0.77 mg, 0.79 x 10⁻⁶ mol, S/C 5'000), DBU (30.0 mg, 1.97 x 10⁻⁴ mol, S/B 20) and EtOH (20 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar for 23 h. Reaction samples were taken at different time points (see *Table 16*) to follow the progress of the reaction.

**Table 16:**

| rct | conv | **3** | | *trans-***4** | *trans-***5** | **6** | *(R,R)*-**6** | **6** |
|---|---|---|---|---|---|---|---|---|
| [h] | [%] | [area-%] | [%ee] | [arca-%] | [area-%] | [area-%] | [‰ee] | [*trans*/*cis*] |
| 1 | 22 | 19 | - | - | - | - | - | - |
| 2 | 88 | 85 | 94.3 | 1.6 | - | - | - | - |
| 4 | 97 | 77 | 93.6 | 11 | 4.2 | - | - | - |
| 6 | >99.9 | 9 | - | 54 | 30 | 5 | - | - |
| 23 | >99.9 | - | - | 1.7 | 0.4 | 96.2 | >99.9 | 24 |

### Example 2.59

In a glove box under argon atmosphere, a 380 mL autoclave was charged with **1** (15.0 g, 59 mmol), **630** (45.1 mg, 5.9 x 10⁻⁵ mol, S/C 1'000), **680** (11.5 mg, 1.2 x 10⁻⁵ mol, S/C 5'000), DBU (179.3 mg, 1.2 mmol, S/B 50) and EtOH (300 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar. Reaction samples were taken at different time points (see *Table 17)* to follow the progress of the reaction. After a total reaction time of 48 h (>99.9% conversion), the autoclave was vented and allowed to cool to room temperature. The reaction mixture was transferred with aid of EtOH (200 mL) from the autoclave into a 500 mL round bottomed flask and the orange reaction solution rotatory evaporated at 40°C / 10 mbar to constant weight to afford crude **6** (12.1 g - a higher yield would be achievable when omitting IPC sampling) with 99.4 area-% purity and a *trans*/*cis* ratio of 15. (*R,R*)-**6** was obtained with >99.9% ee.

### Specified impurities: cis-6 (6.1%), trans-4 (0.2 %), trans-5 (0.1%)

Next, crude (*R,R*)-**6** (12.1 g) was suspended in iPrOAc (100 mL) and the slurry stirred for 2h at 50°C. The suspension was cooled to 0°C and stirred at this temperature for 1h, filtered and the filter cake washed with ice-cold iPrOAc (60 ml) in 3 portions to afford after drying (25°C, 10 mbar) pure **6** (9.8 g, 77% yield) with 99.5 area-% purity and a *trans*/*cis* ratio of 104. (*R,R*)-**6** was obtained with >99.9% ee.

### Specified impurity: cis-6 (0.95%)

Subsequently, (*R,R*)-**6** (9.8 g) from above was dissolved in iPrOAc (78 ml) at 90°C. The colorless solution was cooled to 25°C within 2 h whereby the product started to crystallize. The formed suspension was kept at 25°C for 2 h and cooled to 0°C within 30 min. The crystals were filtered and washed with ice-cold iPrOAc (30 ml) in 2 portions to afford after drying (25°C, 10 mbar) off-white, crystalline **6** (9.0 g, 71% yield - a higher yield would be achievable when omitting IPC sampling during the hydrogenation run) with >99.9 area-% purity and a *trans*/*cis* ratio of 713. (*R,R*)-**6** was obtained with >99.9% ee.

### Specified impurity: cis-6 (0.14%)

**Table 17:**

| rct | conv | **3** | | *trans-***4** | *trans-***5** | **6** | *(R,R)-***6** | **6** |
|---|---|---|---|---|---|---|---|---|
| [h] | [%] | [area-%] | [%ee] | [area-%] | [area-%] | [area-%] | [%ee] | [*trans*/*cis*] |
| 2 | 89 | 88 | 94 | 0.4 | 0 | 0 | - | - |
| 4 | >99.9 | 85 | 93 | 11 | 0 | 0.1 | - | - |
| 6 | >99.9 | 34 | 92 | 44 | 19 | 0.4 | - | - |
| 20 | >99.9 | 0 | - | 0.9 | 0.2 | 96.4 | >99.9 | 18 |
| 23 | >99.9 | 0 | - | 0.7 | 0.1 | 97.0 | >99.9 | 18 |
| 46 | >99.9 | 0 | - | 0.2 | 0.1 | 96.8 | >99.9 | 15 |

### Example 2.60

In a glove box under argon atmosphere, a 380 mL autoclave was charged with **1** (15.0 g, 59 mmol), **630** (45.1 mg, 5.9 x 10⁻⁵ mol, S/C 1'000), **680** (11.5 mg, 1.2 x 10⁻⁵ mol, S/C 5'000), DBU (179.3 mg, 1.2 mmol, S/B 50) and EtOH (300 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar. Reaction samples were taken at different time points (see *Table 18)* to follow the progress of the reaction. After a total reaction time of 23 h (>99.9% conversion), the autoclave was vented and allowed to cool to room temperature. The reaction mixture was transferred with aid of EtOH (200 mL) from the autoclave into a 500 mL round bottomed flask and the orange reaction solution rotatory evaporated at 40°C / 10 mbar to constant weight to afford crude **6** (12.4 g - a higher yield would be achievable when omitting IPC sampling) with 98.7 area-% purity and a *trans*/*cis* ratio of 14. (*R,R*)-**6** was obtained with >99.9% ee.

### Specified impurities: cis-6 (6.5%), trans-4 (0.2 %), trans-5 (0.4%)

Next, crude (*R,R*)-**6** (12.4 g) was suspended in DCM (100 mL) and the slurry stirred for 2h at 50°C. The suspension was cooled to 0°C and stirred at this temperature for 1 h, filtered and the filter cake washed with ice-cold DCM (60 ml) in 3 portions to afford after drying (25°C, 10 mbar) pure **6** (11.0 g, 91% yield) with 99.8 area-% purity and a *trans*/*cis* ratio of 65. (*R,R*)-**6** was obtained with >99.9% ee.

### Specified impurity: cis-6 (1.52%)

Subsequently, (*R,R*)-**6** (11.0 g) from above was dissolved in iPrOAc (88 ml) at 90°C. The colorless solution was cooled to 25°C within 2h whereby the product started to crystallize. The formed suspension was kept at 25°C for 2 h and cooled to 0°C within 30 min. The crystals were filtered and washed with ice-cold iPrOAc (30 ml) in 2 portions to afford after drying (25°C, 10 mbar) off white, crystalline **6** (9.0 g, 75% yield - a higher yield would be achievable when omitting IPC sampling during the hydrogenation run) with >99.9 area-% purity and a *trans*/*cis* ratio of 713. (*R,R*)-**6** was obtained with >99.9% ee.

### Specified impurity: cis-6 (0.14%)

**Table 18:**

| rct | conv | **3** | | *trans*-**4** | *trans*-**5** | **6** | *(R,R)-***6** | **6** |
|---|---|---|---|---|---|---|---|---|
| [h] | [%] | [area-%] | [%ee] | [area-%] | [area-%] | [area-%] | [%ee] | [*trans*/*cis*] |
| 2 | 89 | 87 | 95 | 0.4 | 0 | 0 | - | - |
| 4 | >99.9 | 95 | 93 | 4.5 | 0.1 | 0.2 | - | - |
| 23 | >99.9 | - | - | 0.2 | 0.4 | 98.1 | >99.9 | 14 |

### Example 2.61

In a glove box under argon atmosphere, a 380 mL autoclave was charged with **1** (15.0 g, 59 mmol), **630** (45.1 mg, 5.9 x 10⁻⁵ mol, S/C 1'000), **680** (11.5 mg, 1.2 x 10⁻⁵ mol, S/C 5'000), DBU (179.3 mg, 1.2 mmol, S/B 50) and EtOH (300 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar. After a total reaction time of 23 h (>99.9% conversion), the autoclave was vented and allowed to cool to room temperature. The reaction mixture was transferred with aid of EtOH (200 mL) from the autoclave into a 500 mL round bottomed flask and the orange reaction solution rotatory evaporated at 40°C / 10 mbar to constant weight to afford crude **6** (13.2 g) with 99.4 area-% purity and a *trans*/*cis* ratio of 18. (*R,R*)-**6** was obtained with >99.9% ee.

### Specified impurities: cis-6 (5.1%), trans-5 (0.3%)

Next, crude (*R,R*)-**6** (13.2 g) was suspended in iPrOAc (106 mL) and the slurry stirred for 2h at 50°C. The suspension was cooled to 0°C and stirred at this temperature for 1h, filtered and the filter cake washed with ice-cold iPrOAc (60 ml) in 3 portions to afford after drying (25°C, 10 mbar) pure **6** (10.6 g, 83% yield) with 99.8 area-% purity and a *trans*/*cis* ratio of 91. (*R,R*)**-6** was obtained with >99.9% ee.

### Specified impurity: cis-6 (1.1%)

Subsequently, (*R*,*R*)-**6** (10.6 g) from above was dissolved in iPrOAc (85 ml) at 90°C. The colorless solution was cooled to 25°C within 2 h whereby the product started to crystallize. The formed suspension was kept at 25°C for 2 h and cooled to 0°C within 30 min. The crystals were filtered and washed with ice-cold iPrOAc (30 ml) in 2 portions to afford after drying (25°C, 10 mbar) off-white, crystalline **6** (9.8 g, 77% yield) with >99.9 area-% purity and a *trans*/*cis* ratio of 249. (*R,R*)-**6** was obtained with >99.9% ee.

### Specified impurity: cis-6 (0.41%)

### Analytical data for trans-6

GC-MS ESI (m/z): 216.0 [M+]
**NMR** (400 MHz, DMSO) δ 7.27 - 7.42 (m, 4H), 5.21 (d, *J* = 4.8 Hz, 1H), 4.69 - 4.82 (m, 1H), 4.48 (br d, J=4.6 Hz, 2H), 3.62 - 3.75 (m, 1H), 3.20 - 3.31 (m, 2H), 1.59 - 1.73 (m, 1H), 1.42 (ddd, J=13.9, 9.5, 2.2Hz, 1H).

### 3. Preparation of (R,R)-6 via asymmetric hydrogenation of (R)-3

### Example 3.1

In a glove box under argon atmosphere, a 185 mL autoclave was charged with (*R*)-**3** (1.00 g, 3.91 mmol, quality: 99.9% ee, 99.8 area-% purity), **680** (3.83 mg, 3.91 x 10⁻⁶ mol, S/C 1'000) and DBU (59.5 mg, 3.91 x 10⁻⁴ mol, S/B 10) and EtOH (20 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar. Reaction samples were taken at different time points (see *Table 19)* to follow the progress of the reaction.

**Table 19:**

| rct | conv | *trans-***4** | *trans-***5** | **6** | *(R,R)*-**6** | **6** |
|---|---|---|---|---|---|---|
| [h] | [%] | [area-%] | [area-%] | [area-%] | [%ee] | [*trans*/*cis*] |
| 1 | 46 | 14 | 7 | 0.8 | n.d. | - |
| 2 | 99.2 | 19 | 19 | 54 | 99.9 | 84 |
| 3 | >99.9 | 0.4 | 1.1 | 94 | 99.9 | 49 |
| 4 | >99.9 | 0 | 0.2 | 95 | 99.9 | 40 |
| 5 | >99.9 | 0 | 0 | 95.5 | 99.9 | 37 |
| 6 | >99.9 | 0 | 95.7 | 95.7 | 99.9 | 38 |

### Example 3.2 - 3.3

In analogy to *Example 3.1*, **3** (0.25 g, 0.98 mmol) was hydrogenated in the presence of **680** (0.19 mg, 0.20 x 10⁻⁶ mol, S/C 5'000) for 23 h in EtOH (4 mL) at 30°C and the presence of DBU as base in amounts as listed in *Table 20.*

**Table 20:**

| Exp | base | S/B | conv | *trans-***4** | *trans-***5** | **6** | *(R,R)***-6** | **6** |
|---|---|---|---|---|---|---|---|---|
| | | | [%] | [area-%] | [area-%] | [area-%] | [%ee] | [*trans*/*cis*] |
| 3.2 | DBU | 20 | >99.9 | 0.3 | 0.1 | 94 | >99.9 | 39 |
| 3.3 | DBU | 50 | >99.9 | 0.1 | 0.1 | 97.2 | >99.9 | 36 |

### Example 3.4

In a glove box under argon atmosphere, a 185 mL autoclave was charged with (*R*)-**3** (6.0 g, 23.0 mmol, quality: 99.9% ee, 99.8 area-% purity) **680** (22.9 mg, 2.3 x 10⁻⁵ mol, S/C 1'000) and DBU (71.2 mg, 4.7 x 10⁻⁴ mol, S/B 50) and EtOH (120 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar. After a total reaction time of 23 h (99.9% conversion), the autoclave was vented and allowed to cool to room temperature. The reaction mixture was transferred with aid of EtOH (20 mL) from the autoclave into a 250 mL round bottomed flask and the orange reaction solution rotatory evaporated at 40°C / 10 mbar to constant weight to yield crude (*R,R*)-**6** (5.2 g) with 98.7 area-% purity and a *trans*/*cis* ratio of 28. (*R,R*)-**6** was obtained with >99.9% ee.

### Specified impurities: cis-6 (3.40%), 3 (0.10%)

Next, crude (*R,R*)-**6** (5.2 g) was suspended in iPrOAc (52 mL) and the slurry stirred for 2h at 50°C. The suspension was cooled to 0°C and stirred at this temperature for 1h, filtered and the filter cake washed with ice-cold iPrOAc (30 ml) in 3 portions to afford after drying (25°C, 10 mbar) pure **6** (4.1 g, 81% yield) with 99.5 area-% purity and a *trans*/*cis* ratio of 125. (*R,R*)-**6** was obtained with >99.9% ee.

### Specified impurity: cis-6 (0.79%)

Subsequently, (*R,R*)-**6** (4.1 g) from above was dissolved in iPrOAc (34 ml) at 90°C. The colorless solution was cooled to 25°C within 2 h whereby the product started to crystallize. The formed suspension was kept at 25°C for 2 h and cooled to 0°C within 30 min. The crystals were filtered and washed with ice-cold iPrOAc (14 ml) in 2 portions to afford after drying (25°C, 10 mbar) off white, crystalline **6** (3.7 g, 73% yield) with >99.9 area-% purity and a *trans*/*cis* ratio of 586. (*R,R*)-**6** was obtained with >99.9% ee.

### Specified impurity: cis-6 (0.17%)

### 4. Preparation of (R,R)-6 via asymmetric hydrogenation of (R,R)-5

### Example 4.1

In a glove box under argon atmosphere, a 185 mL autoclave was charged with (*R,R*)-**5** (1.00 g, 4.71 mmol; quality: 99.9% ee, 99.9 area-% purity), **680** (4.62 mg, 4.71 x 10⁻⁶ mol, S/C 1'000), DBU (71.7 mg, 4.71 x 10⁻⁴ mol, S/B 10) and EtOH (20 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar. A reaction sample was taken at different time points (see *Table 21*) to follow the progress of the reaction.

**Table 21:**

| rct | conv | **6** | *(R,R)-**6*** | **6** |
|---|---|---|---|---|
| [h] | [%] | [area-%] | [%ee] | [*trans*/*cis*] |
| 1 | 90 | 82 | 99.9 | >100 |
| 2 | >99.9 | 98.2 | 99.9 | >100 |

### Example 4.2

In analogy to Example 4.1, *(R,R)-***5** (0.25 g, 1.18 mmol; quality: 99.9% ee, 99.9 area-% purity) was hydrogenated in the presence of **680** (0.23 mg, 2.36 x 10⁻⁷ mol, S/C 5'000) and DBU (9.0 mg, 0.59 x 10⁻⁴ mol, S/B 20) in EtOH (5 mL) to yield after 20 h at 30°C and an initial hydrogen pressure of 70 bar crude (*R,R*)-**6** with 96.7% purity and >99.9% ee (99% conversion; *trans*/*cis* ratio >100)

### 5. Preparation of (R,R)-8 via asymmetric hydrogenation of 7

### Examples 5.1

In a glove box under argon atmosphere, a 35 mL autoclave was charged with **7** (250 mg, 1.1 mmol), **680** (1.1 mg, 1.1 x 10⁻⁶ mol, S/C 1'000), KOtBu (12.1 mg, 1.1 x 10⁻⁴ mol, S/B 10) and EtOH (5 mL). The autoclave was sealed and removed from the glove box, connected to a hydrogen line and pressurized with hydrogen gas to 70 bar and heated to 30°C. Under stirring, the hydrogenation was ran at a constant hydrogen pressure of 70 bar. After a total reaction time of 20 h, the autoclave was vented and allowed to cool to room temperature. The reaction mixture was transferred with aid of EtOH (5 mL) from the autoclave into a 50 mL round bottomed flask and the orange reaction solution rotatory evaporated at 40°C / 10 mbar to constant weight to yield crude *trans-***8** (presumable major enantiomer: (*R,R*)-**8**, 245 mg) with >95% LC/MS purity.

### Analytical data for trans-8

GC-MS ESI (m/z): 182.1 [M+]
¹H-NMR (CDCl₃, 600 MHz): δ ppm 7.30 - 7.42 (m, 1H), 7.28 - 7.42 (m, 3H), 5.03 (br d, J=3.8 Hz, 1H), 3.98 (br s, 1H), 3.44 - 3.64 (m, 2H), 3.03 - 3.44 (m, 2H), 2.38 - 2.86 (m, 1H), 1.72 - 2.03 (m, 3H)
¹³C-NMR (CDCl₃, 151 MHz): δ ppm 144.2, 128.5, 127.5, 125.5, 71.4, 69.4, 66.8, 41.0 ppm.

## Claims

1. Process for the preparation of a chiral triol of formula I 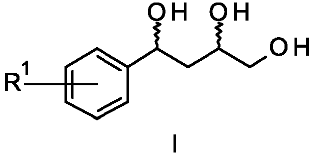 wherein
R¹ is hydrogen or halogen and
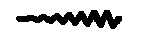 denotes either a dashed bond (a) or a wedged bond (b)
a) 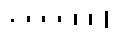 b) 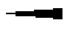 .
comprising the asymmetric hydrogenation of a ketone compound of formula IIa 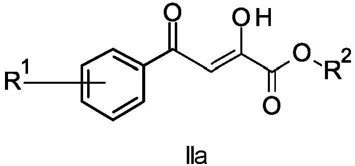 wherein
R¹ is hydrogen or halogen and
R² is
with hydrogen in the presence of an iridium spiro-pyridylamidophosphine catalyst (Ir-SpiroPAP catalyst) of the formula IIIa or IIIb, or enantiomers thereof, 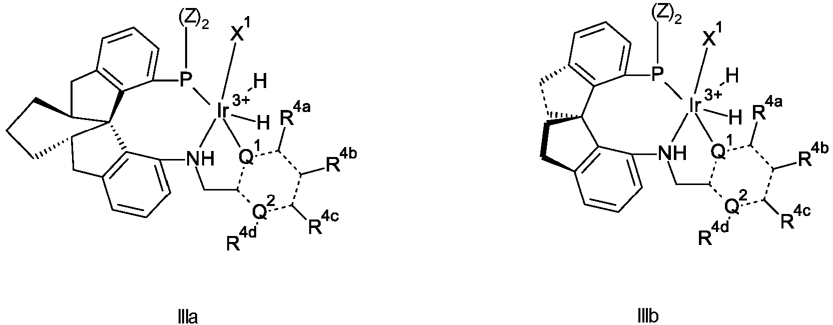 wherein
R^{4a}, R^{4b}, R^{4c} and R^{4d} independently of each other are hydrogen or C₁₋₆-alkyl; the dotted ring signifies an aromatic ring when Q¹ is nitrogen and Q² is carbon and the dotted ring signifies a cycloalkane ring wherein Q¹ and Q² are sulfur;
X¹ is either a coordinated ligand or a counter anion selected from halogen, C₁₋₆-alkoxy, tetrahalogeno borate, hexahalogenoborate, tetrakis(3,5-bis(trihalogeno-C₁₋6-alkyl)phenyl)borate, acetylacetonate, hexahalogenophosphate, p-tolylsulfonate (OTs) or trihalogeno methanesulfonate and
Z is phenyl, optionally substituted by one or more groups selected from C₁₋₈-alkyl, C₁₋₈-halogenalkyl or phenyl; C₃₋₈-cycloalkyl, optionally substituted by one or more C₁₋₈-alkyl groups or di- C₁₋₈-alkyl phosphinyl.

2. Process of claim 1, wherein the Ir-SpiroPAP catalyst is selected from the compounds IIIa or IIIb, or enantiomers thereof,
wherein
R^{4a}, R^{4b}, R^{4c} and R^{4d} independently of each other are hydrogen or C₁₋₄-alkyl;
the dotted ring signifies an aromatic ring when Q¹ is nitrogen and Q² is carbon and the dotted ring signifies a cycloalkane ring wherein Q¹ and Q² are sulfur;
X¹ is either a coordinated ligand or a counter anion selected from halogen, methoxy, tetrafluoroborate (BF4), hexafluoroborate (BF6), tetrakis(3,5-bis(trifluoromethyl) phenyl)borate (barf), acetylacetonate (acac), hexafluorophosphate (PF6), p-tolylsulfonate (OTs) or trifluoromethanesulfonate (OTf) and;
Z is phenyl, optionally substituted by one or more groups selected from C₁₋₆-alkyl, C₁₋₄-halogenalkyl or phenyl or is C₄₋₇-cycloalkyl.

3. Process of claim 1 or 2, wherein the Ir-SpiroPAP catalyst is selected from the compounds IIIa or IIIb, or enantiomers thereof
R^{4a}, R^{4b}, R^{4c} and R^{4d} independently of each other are hydrogen or C₁₋₄-alkyl; the dotted ring signifies an aromatic ring when Q¹ is nitrogen and Q² is carbon and the dotted ring signifies a cycloalkane ring wherein Q¹ and Q² are sulfur;
X¹ is halogen;
Z is phenyl, optionally substituted by one or two groups selected from C₁₋₆-alkyl, C₁₋₄-halogenalkyl or phenyl or is cyclopentyl or cyclohexyl.

4. Process of anyone of claims 1 to 3, wherein the Ir-SpiroPAP catalyst is selected from the compounds 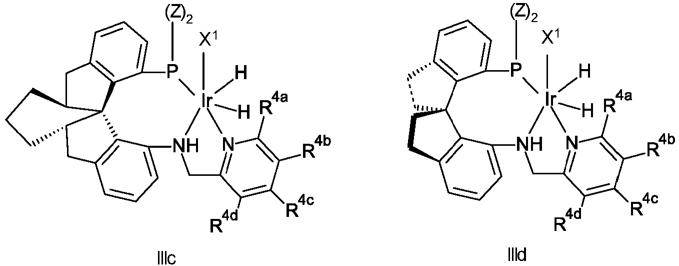 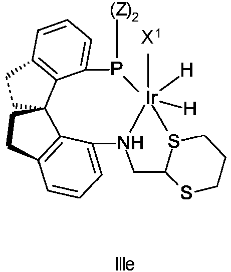 wherein;
R^{4a}, R^{4b}, R^{4c} and R^{4d} independently of each other are hydrogen or C₁₋₄-alkyl;
X¹ is halogen;
Z is phenyl optionally substituted by one or two groups selected from C₁₋₆-alkyl, C₁₋₄-halogenalkyl or phenyl or is cyclopentyl or cyclohexyl.

5. Process of anyone of claims 1 to 4, wherein the asymmetric hydrogenation is performed in the presence of an organic solvent and a base at a hydrogen pressure of 5 bar to 100 bar and at a reaction temperature of 10°C to 90°C.

6. Process of anyone of claims 1 to 5, wherein the organic solvent is an aliphatic alcohol, a halogen substituted alcohol, an ether or an aromatic solvent or is a mixture thereof.

7. Process of anyone of claims 1 to 6, wherein the base is an inorganic base selected from alkali or earth alkali- carbonates or - hydrogen carbonates or phosphates or hydrogenphosphates or dihydrogenphosphates or acetates or formates or organic bases selected from amines, alkali alcoholates or amidines.

8. Process of anyone of claim 1 to 6, wherein the substrate to catalyst ratio is selected in a range of 100 to 10'000.

9. Process of anyone claim 1 to 8, wherein the Ir-SpiroPAP catalyst of formula IIIa or IIIb is prepared in situ in the course of the asymmetric hydrogenation reaction by bringing together a Iridium-pre catalyst complex with a spiro-pyridylamidophosphine ligand of the formula 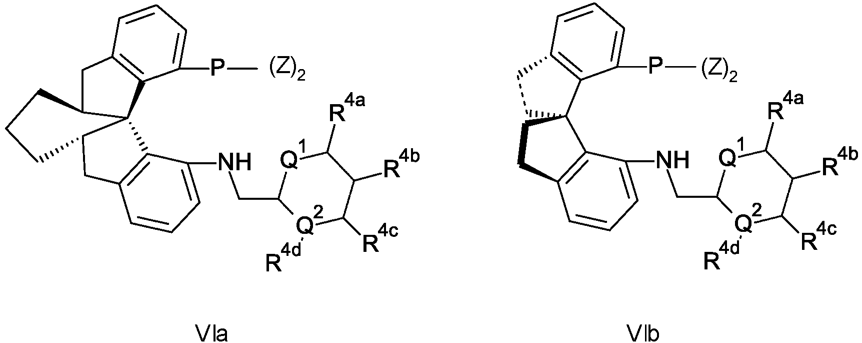 wherein R^{4a}, R^{4b}, R^{4c} and R^{4d}, Q¹ and Q² and Z have the meanings as outlined above.

10. Process of claim 9, wherein the Iridium-pre catalyst complex is selected from [Ir(cod)₂]BF₄, [IrCl(COD)]₂, [Ir(acac)(COD)], [Ir(OMe)(COD)]₂, [Ir(cod)₂]BARF, [Ir(cod)₂]PF6.

11. Process of claim 1, wherein the asymmetric hydrogenation of the ketone of formula IIa in a first step is performed in the presence of the Ir-PEN catalyst of formula IVa or IVb, or enantiomers thereof, 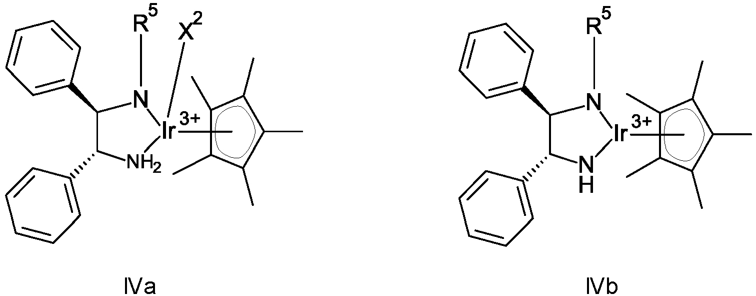 wherein,
R⁵ is C₁₋₆-alkylsulfonyl wherein the alkyl group is optionally substituted with one or more halogen atoms; with a 7,7-dimethyl-2-oxobicyclo[2.2.1] heptane-1-yl group or phenyl sulfonyl, wherein the phenyl group is optionally substituted by one or more C₁₋₆-alkyl groups and
X² is either a coordinated ligand or a counter anion selected from a C₁₋₆-alkylsulfonyloxy group which is optionally substituted with one or more halogen, atoms; from halogen, C₁₋₆-alkoxy, tetrahalogenoborate, hexahalogenoborate, tetrakis(3,5-bis(trihalogeno-C₁₋₆-alkyl)phenyl)borate, acetylacetonate, hexahalogenophosphine, p-tolylsulfonate (OTs) or trihalogenomethanesulfonate;
to form the ketone of formula IIb, 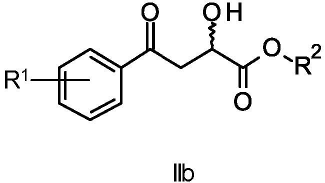 wherein R¹ and R² are as above,
and in a subsequent step the ketone of formula IIb is further subjected to an asymmetric hydrogenation in the presence of an Ir-SpiroPAP catalyst of the formula IIIa or IIIb, or enantiomers thereof, to form the chiral triol of formula I.

12. Process of claim 11, wherein
R⁵ is methylsulfonyl, trifluoromethylsulfonyl, 7,7-dimethyl-2-oxobicyclo[2.2.1] heptane-1-yl; tolylsulfonyl or 1,3,5-tri-i-propylphenyl sulfonyl;
X² is either a coordinated ligand or a counter anion selected from a methylsulfonyloxy group which is optionally substituted with one or more fluoro atoms; from halogen, methoxy, tetrafluoroborate (BF4), hexafluoroborate (BF6), tetrakis(3,5-bis(trifluoromethyl)phenyl)borate (barf), acetylacetonate (acac), hexafluorophosphine (PF6), p-tolylsulfonate (OTs) or trifluoromethanesulfonate (OTf.

13. Process of claim 11 or 12, wherein the iridium-phenylendiamine catalyst (Ir-PEN catalyst) are of the formula IVa, or enantiomers thereof, wherein,
R⁵ is methylsulfonyl, trifluoromethylsulfonyl, 7,7-dimethyl-2-oxobicyclo[2.2.1] heptane-1-yl; tolylsulfonyl or 1,3,5-tri-i-propylphenyl sulfonyl;
X² is a trifluoromethylsulfonyl oxy group; or
are of the formula IVb, or enantiomers thereof, wherein,
R⁵ is methylsulfonyl, trifluoromethylsulfonyl, 7,7-dimethyl-2-oxobicyclo[2.2.1] heptane-1-yl; tolylsulfonyl or 1,3,5-tri-i-propylphenyl sulfonyl.

14. Process of anyone of claims 11 to 13, wherein the asymmetric hydrogenation of the ketone of formula IIa is performed in the presence of an organic solvent at a hydrogen pressure of 5 bar to 100 bar and at a reaction temperature of 10°C to 90°C.

15. Process of claim 14, wherein the organic solvent is an aliphatic alcohol, a halogen substituted alcohol, an ether or an aromatic solvent or is a mixture thereof.

16. Process of claims 14 or 15, wherein the substrate to catalyst ratio is selected in a range of 100 to 1000.

17. Process of anyone of claims 1 to 16, wherein the asymmetric hydrogenation of the ketone of formula IIa takes place in the presence of a mixture of an Ir-Spiro PAP catalyst of the formula IIIa or IIIb, or of an enantiomer thereof, and an Ir-PEN catalyst of the formula IVa or IVb, or of an enantiomer thereof.

18. Process of claim 17 wherein the reaction is performed in the presence of an organic solvent and a base at a hydrogen pressure of 5 bar to 100 bar and at a reaction temperature of 10°C to 90°C.

19. Process of claims 17 or 18, wherein the organic solvent is an aliphatic alcohol, a halogen substituted alcohol, an ether or an aromatic solvent or is a mixture thereof.

20. Process of any one of claims 17 to 19, wherein the base is an inorganic base selected from alkali or earth alkali- carbonates or - hydrogen carbonates or phosphates or hydrogenphosphates or dihydrogenphosphates or acetates or formiates or organic bases selected from amines, alkali alcoholates or amidines.

21. Process of any one of claims 17 to 20, wherein the substrate to Ir-PEN catalyst ratio is selected in a range of 100 to 10000, preferably in a range of 500 to 1000 and the substrate to Ir-Spiro PAP catalyst ratio is selected in a range of 100 to 10000, preferably in a range of 2500 to 7500.

22. Process of claim 1, wherein the intermediates in the asymmetric hydrogenation of the ketone of formula IIa to the chiral triol of formula I of the formula 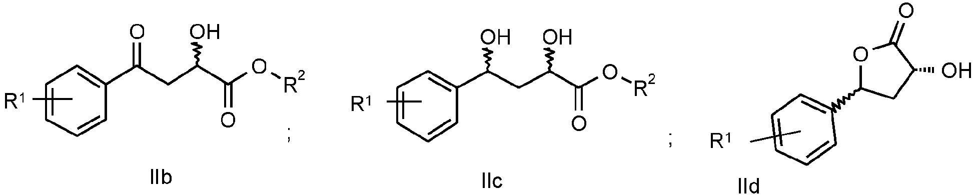 wherein R¹ and R² are as above, are individually isolated and individually be subjected to the asymmetric hydrogenation in the presence of an Ir-Spiro PAP catalyst of the formula IIIa or IIIb.

23. Process of any one of claims 1 to 22, wherein the chiral triol has the formula Ia 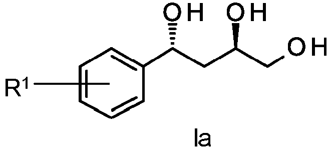 R¹ is hydrogen or halogen.

24. Process of claim 23, wherein R¹ is halogen.

25. Process of any one of claims 1 to 24, wherein the chiral triol has the formula Ib

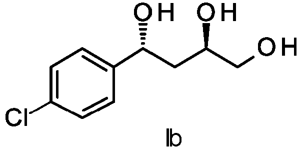

## Patentansprüche

1. Verfahren zur Herstellung eines chiralen Triols der Formel I 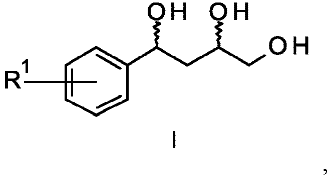 wobei
R¹ Wasserstoff oder Halogen ist und
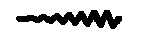 entweder eine gestrichelte Bindung (a) oder eine keilförmige Bindung (b) kennzeichnet
a) 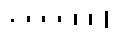 b) 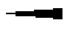 ,
umfassend die asymmetrische Hydrierung einer Ketonverbindung der Formel IIa 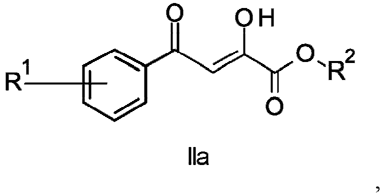 wobei
R¹ Wasserstoff oder Halogen ist und
R² C₁₋₆-Alkyl ist;
mit Wasserstoff in der Gegenwart eines Iridiumspiro-pyridylamidophosphin-Katalysators (Ir-SpiroPAP-Katalysator) der Formel IIIa oder IIIb oder Enantiomeren davon 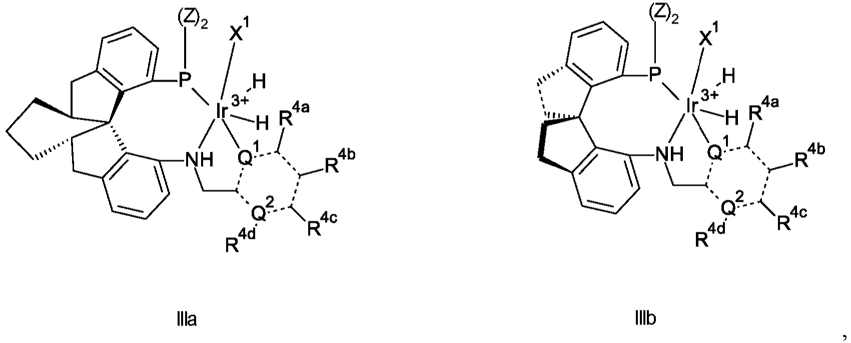 wobei
R^{4a}, R^{4b}, R^{4c} und R^{4d} unabhängig voneinander Wasserstoff oder C₁₋₆-Alkyl sind;
der gepunktete Ring einen aromatischen Ring bedeutet, wenn Q¹ Stickstoff ist und Q² Kohlenstoff ist, und der gepunktete Ring einen Cycloalkanring bedeutet, wobei Q¹ und Q² Schwefel sind;
X¹ entweder ein koordinierter Ligand oder ein Gegenanion ist, ausgewählt aus Halogen, C₁₋₆-Alkoxy, Tetrahalogenborat, Hexahalogenborat, Tetrakis(3,5-bis(trihalogen-C₁₋₆-alkyl)phenyl)borat, Acetylacetonat, Hexahalogenphosphat, p-Tolylsulfonat (OTs) oder Trihalogenmethansulfonat, und
Z Phenyl, das gegebenenfalls mit einer oder mehreren Gruppe(n) substituiert ist, die aus C₁₋₈-Alkyl, C₁₋₈-Halogenalkyl oder Phenyl ausgewählt sind; C₃₋₈-Cycloalkyl, das gegebenenfalls mit einer oder mehreren C₁₋₈-Alkylgruppen substituiert ist, oder Di-C₁₋₈-alkylphosphinyl ist.

2. Verfahren nach Anspruch 1, wobei der Ir-SpiroPAP-Katalysator aus den Verbindungen IIIa oder IIIb oder Enantiomeren davon ausgewählt ist,
wobei
R^{4a}, R^{4b}, R^{4c} und R^{4d} unabhängig voneinander Wasserstoff oder C₁₋₄-Alkyl sind;
der gepunktete Ring einen aromatischen Ring bedeutet, wenn Q¹ Stickstoff ist und Q² Kohlenstoff ist, und der gepunktete Ring einen Cycloalkanring bedeutet, wobei Q¹ und Q² Schwefel sind;
X¹ entweder ein koordinierter Ligand oder ein Gegenanion ist, ausgewählt aus Halogen, Methoxy, Tetrafluorborat (BF₄), Hexafluorborat (BF₆), Tetrakis(3,5-bis(trifluormethyl)phenyl)borat (barf), Acetylacetonat (acac), Hexafluorphosphat (PF₆), p-Tolylsulfonat (OTs) oder Trifluormethansulfonat (OTf), und
Z Phenyl, das gegebenenfalls mit einer oder mehreren Gruppe(n) substituiert ist, die aus C₁₋₆-Alkyl, C₁₋₄-Halogenalkyl oder Phenyl ausgewählt sind, oder C₄₋₇-Cycloalkyl ist.

3. Verfahren nach Anspruch 1 oder 2, wobei der Ir-SpiroPAP-Katalysator aus den Verbindungen IIIa oder IIIb oder Enantiomeren davon ausgewählt ist,
R^{4a}, R^{4b}, R^{4c} und R^{4d} unabhängig voneinander Wasserstoff oder C₁₋₄-Alkyl sind;
der gepunktete Ring einen aromatischen Ring bedeutet, wenn Q¹ Stickstoff ist und Q² Kohlenstoff ist, und der gepunktete Ring einen Cycloalkanring bedeutet, wobei Q¹ und Q² Schwefel sind;
X¹ Halogen ist;
Z Phenyl, das gegebenenfalls mit einer oder zwei Gruppe(n) substituiert ist, die aus C₁₋₆-Alkyl, C₁₋₄-Halogenalkyl oder Phenyl ausgewählt sind, oder Cyclopentyl oder Cyclohexyl ist.

4. Verfahren nach einem der Ansprüche 1 bis 3, wobei der Ir-SpiroPAP-Katalysator aus den folgenden Verbindungen ausgewählt ist 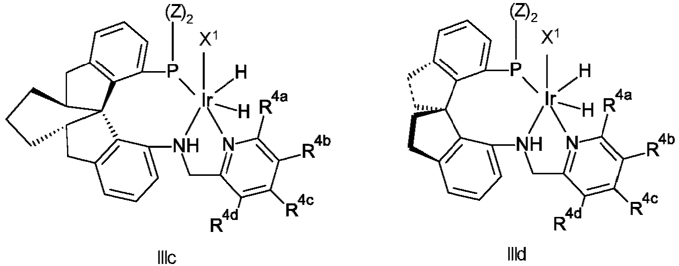 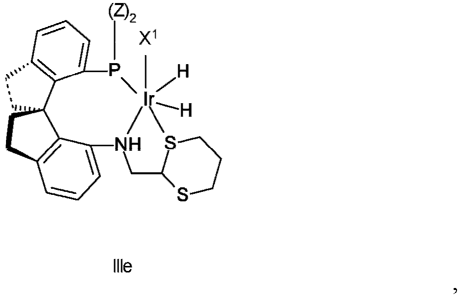 wobei
R^{4a}, R^{4b}, R^{4c} und R^{4d} unabhängig voneinander Wasserstoff oder C₁₋₄-Alkyl sind;
X¹ Halogen ist;
Z Phenyl, das gegebenenfalls mit einer oder zwei Gruppe(n) substituiert ist, die aus C₁₋₆-Alkyl, C₁₋₄-Halogenalkyl oder Phenyl ausgewählt sind, oder Cyclopentyl oder Cyclohexyl ist.

5. Verfahren nach einem der Ansprüche 1 bis 4, wobei die asymmetrische Hydrierung in der Gegenwart eines organischen Lösungsmittels und einer Base bei einem Wasserstoffdruck von 5 bar bis 100 bar und bei einer Reaktionstemperatur von 10 °C bis 90 °C durchgeführt wird.

6. Verfahren nach einem der Ansprüche 1 bis 5, wobei das organische Lösungsmittel ein aliphatischer Alkohol, ein halogensubstituierter Alkohol, ein Ether oder ein aromatisches Lösungsmittel oder ein Gemisch davon ist.

7. Verfahren nach einem der Ansprüche 1 bis 6, wobei die Base eine anorganische Base, die aus Alkali- oder Erdalkalicarbonaten oder -hydrogencarbonaten oder -phosphaten oder -hydrogenphosphaten oder -dihydrogenphosphaten oder -acetaten oder -formiaten ausgewählt ist, oder organische Basen, die aus Aminen, Alkalialkoholaten oder Amidinen ausgewählt sind, ist.

8. Verfahren nach einem der Ansprüche 1 bis 6, wobei das Substrat-zu-Katalysator-Verhältnis aus einem Bereich von 100 bis 10.000 ausgewählt ist.

9. Verfahren nach einem der Ansprüche 1 bis 8, wobei der Ir-SpiroPAP-Katalysator der Formel IIIa oder IIIb in situ während der asymmetrischen Hydrierungsreaktion hergestellt wird, indem ein Iridium-Präkatalysator-Komplex mit einem Spiro-Pyridylamidophosphin-Liganden der folgenden Formel zusammengebracht wird 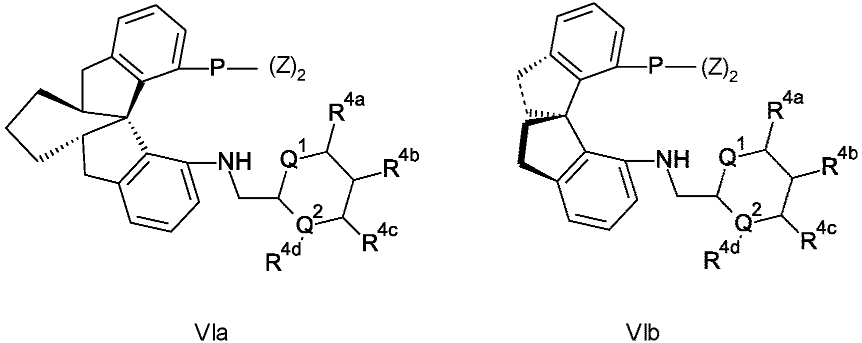 , wobei R^{4a}, R^{4b}, R^{4c} und R^{4d}, Q¹ und Q² und Z die oben aufgeführten Bedeutungen haben.

10. Verfahren nach Anspruch 9, wobei der Iridium-Präkatalysator-Komplex aus [Ir(cod)₂]BF₄, [IrCl(COD)]₂, [Ir(acac)(COD)], [Ir(OMe)(COD)]₂, [Ir(cod)₂]BARF, [Ir(cod)₂]PF₆ ausgewählt ist.

11. Verfahren nach Anspruch 1, wobei die asymmetrische Hydrierung des Ketons der Formel IIa in einem ersten Schritt in der Gegenwart des Ir-PEN-Katalysators der Formel IVa oder IVb oder Enantiomeren davon durchgeführt wird 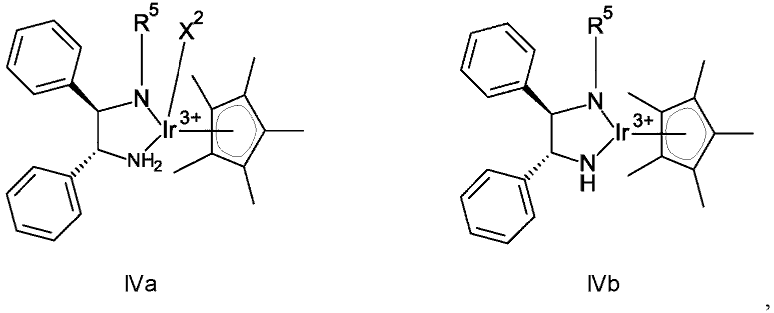 wobei
R⁵ C₁₋₆-Alkylsulfonyl ist, wobei die Alkylgruppe gegebenenfalls mit einem oder mehreren Halogenatom(en), mit einer 7,7-Dimethyl-2-oxobicyclo[2.2.1]heptan-1-yl-Gruppe oder Phenylsulfonyl substituiert ist, wobei die Phenylgruppe gegebenenfalls mit einer oder mehreren C₁₋₆-Alkylgruppe(n) substituiert ist, und
X² entweder ein koordinierter Ligand oder ein Gegenanion ist, ausgewählt aus einer C₁₋₆-Alkylsulfonyloxygruppe, die gegebenenfalls mit einem oder mehreren Halogenatom(en) substituiert ist, aus Halogen, C₁₋₆-Alkoxy, Tetrahalogenborat, Hexahalogenborat, Tetrakis(3,5-bis(trihalogen-C₁₋₆-alkyl)phenyl)borat, Acetylacetonat, Hexahalogenphosphin, p-Tolylsulfonat (OTs) oder Trihalogenmethansulfonat;
wodurch das Keton der Formel IIb gebildet wird 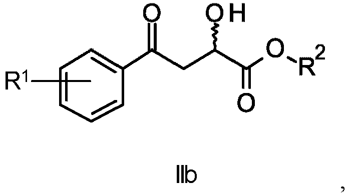 wobei R¹ und R² wie oben sind,
und in einem anschließenden Schritt das Keton der Formel IIb ferner einer asymmetrischen Hydrierung in der Gegenwart eines Ir-SpiroPAP-Katalysators der Formel IIIa oder IIIb oder Enantiomeren davon unterzogen wird, wodurch das chirale Triol der Formel I gebildet wird.

12. Verfahren nach Anspruch 11, wobei
R⁵ Methylsulfonyl, Trifluormethylsulfonyl, 7,7-Dimethyl-2-oxobicyclo[2.2.1]heptan-1-yl, Tolylsulfonyl oder 1,3,5-Tri-i-propylphenylsulfonyl ist;
X² entweder ein koordinierter Ligand oder ein Gegenanion ist, ausgewählt aus einer Methylsulfonyloxygruppe, die gegebenenfalls mit einem oder mehreren Fluoratom(en) substituiert ist; aus Halogen, Methoxy, Tetrafluorborat (BF₄), Hexafluorborat (BF₆), Tetrakis(3,5-bis(trifluormethyl)phenyl)borat (barf), Acetylacetonat (acac), Hexafluorphosphin (PF₆), p-Tolylsulfonat (OTs) oder Trifluormethansulfonat (OTf).

13. Verfahren nach Anspruch 11 oder 12, wobei der Iridium-Phenylendiamin-Katalysator (Ir-PEN-Katalysator) die Formel IVa oder Enantiomere davon aufweist, wobei
R⁵ Methylsulfonyl, Trifluormethylsulfonyl, 7,7-Dimethyl-2-oxobicyclo[2.2.1]heptan-1-yl, Tolylsulfonyl oder 1,3,5-Tri-i-propylphenylsulfonyl ist;
X² eine Trifluormethylsulfonyloxygruppe ist; oder
die Formel IVb oder Enantiomere davon aufweist, wobei
R⁵ Methylsulfonyl, Trifluormethylsulfonyl, 7,7-Dimethyl-2-oxobicyclo[2.2.1]heptan-1-yl, Tolylsulfonyl oder 1,3,5-Tri-i-propylphenylsulfonyl ist.

14. Verfahren nach einem der Ansprüche 11 bis 13, wobei die asymmetrische Hydrierung des Ketons der Formel IIa in der Gegenwart eines organischen Lösungsmittels bei einem Wasserstoffdruck von 5 bar bis 100 bar und bei einer Reaktionstemperatur von 10 °C bis 90 °C durchgeführt wird.

15. Verfahren nach Anspruch 14, wobei das organische Lösungsmittel ein aliphatischer Alkohol, ein halogensubstituierter Alkohol, ein Ether oder ein aromatisches Lösungsmittel oder ein Gemisch davon ist.

16. Verfahren nach Anspruch 14 oder 15, wobei das Substrat-zu-Katalysator-Verhältnis aus einem Bereich von 100 bis 1.000 ausgewählt ist.

17. Verfahren nach einem der Ansprüche 1 bis 16, wobei die asymmetrische Hydrierung des Ketons der Formel IIa in der Gegenwart eines Gemisches aus einem Ir-SpiroPAP-Katalysator der Formel IIIa oder IIIb oder einem Enantiomer davon und einem Ir-PEN-Katalysator der Formel IVa oder IVb oder einem Enantiomer davon stattfindet.

18. Verfahren nach Anspruch 17 wobei die Reaktion in der Gegenwart eines organischen Lösungsmittels und einer Base bei einem Wasserstoffdruck von 5 bar bis 100 bar und bei einer Reaktionstemperatur von 10 °C bis 90 °C durchgeführt wird.

19. Verfahren nach Anspruch 17 oder 18, wobei das organische Lösungsmittel ein aliphatischer Alkohol, ein halogensubstituierter Alkohol, ein Ether oder ein aromatisches Lösungsmittel oder ein Gemisch davon ist.

20. Verfahren nach einem der Ansprüche 17 bis 19, wobei die Base eine anorganische Base, die aus Alkali- oder Erdalkalicarbonaten oder -hydrogencarbonaten oder - phosphaten oder -hydrogenphosphaten oder -dihydrogenphosphaten oder -acetaten oder - formiaten ausgewählt ist, oder organische Basen, die aus Aminen, Alkalialkoholaten oder Amidinen ausgewählt sind, ist.

21. Verfahren nach einem der Ansprüche 17 bis 20, wobei das Substrat-zu-Ir-PEN-Katalysator-Verhältnis aus einem Bereich von 100 bis 10.000, vorzugsweise aus einem Bereich von 500 bis 1.000 ausgewählt ist und das Substrat-zu-Ir-SpiroPAP-Katalysator-Verhältnis aus einem Bereich von 100 bis 10.000, vorzugsweise aus einem Bereich von 2.500 bis 7.500 ausgewählt ist.

22. Verfahren nach Anspruch 1, wobei die Zwischenprodukte bei der asymmetrischen Hydrierung des Ketons der Formel IIa zu dem chiralen Triol der Formel I der folgenden Formel 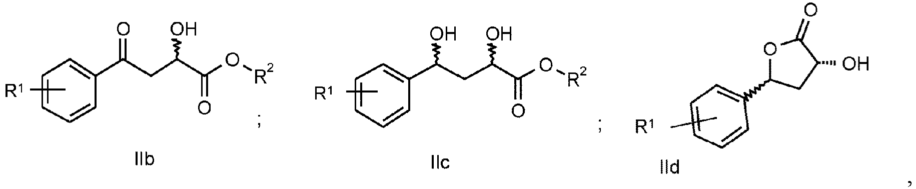 wobei R¹ und R² wie oben sind, einzeln isoliert und einzeln der asymmetrischen Hydrierung in der Gegenwart eines Ir-SpiroPAP-Katalysators der Formel IIIa oder IIIb unterzogen werden.

23. Verfahren nach einem der Ansprüche 1 bis 22, wobei das chirale Triol die Formel Ia aufweist 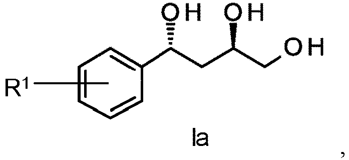 wobei
R¹ Wasserstoff oder Halogen ist.

24. Verfahren nach Anspruch 23, wobei R¹ Halogen ist.

25. Verfahren nach einem der Ansprüche 1 bis 24, wobei das chirale Triol die Formel Ib aufweist

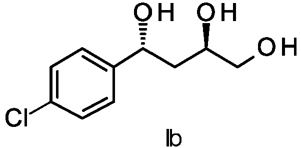

## Revendications

1. Procédé de préparation d'un triol chiral de formule I 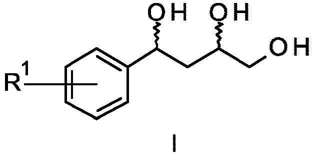 dans lequel
R¹ représente un hydrogène ou un halogène et
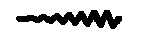 désigne soit une liaison hachurée (a) soit une liaison pleine (b)
a) 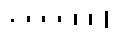 b) 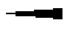 .
comprenant l'hydrogénation asymétrique d'un composé cétone de formule IIa 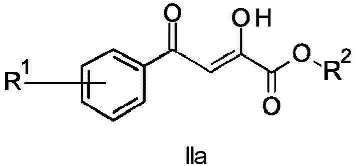 dans lequel
R¹ représente un hydrogène ou un halogène et
R² représente un alkyle en C₁₋₆ ;
avec de l'hydrogène en présence d'un catalyseur spiro-pyridylamidophosphine d'iridium (catalyseur Ir-SpiroPAP) de formule IIIa ou IIIb, ou d'énantiomères de celui-ci, 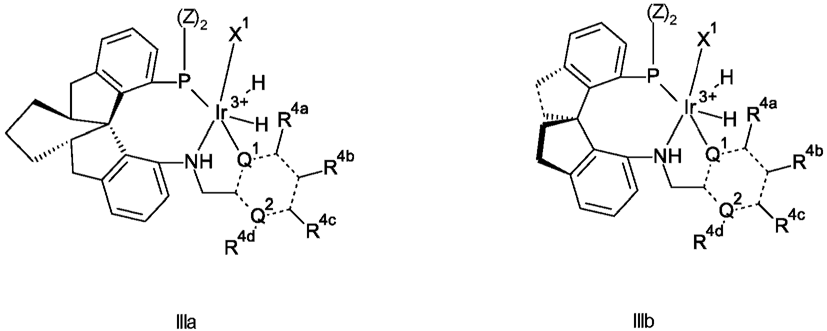 dans lequel
R^{4a}, R^{4b}, R^{4c} et R^{4d} indépendamment les uns des autres représentent un hydrogène ou un alkyle en C₁₋₆ ; ^{c}
le cycle en pointillé signifie un cycle aromatique lorsque Q¹ représente un azote et Q² représente un carbone et le cycle en pointillé signifie un cycle cycloalcane dans lequel Q¹ et Q² représentent un soufre ;
X¹ représente soit un ligand coordonné soit un contre-anion choisi parmi un halogène, un alcoxy en C₁₋₆, un tétrahalogénoborate, un hexahalogénoborate, un tétrakis(3,5-bis(trihalogéno-alkyle en C₁₋₆)phényl)borate, un acétylacétonate, un hexahalogénophosphate, un p-tolylsulfonate (OTs) ou un trihalogénométhanesulfonate et
Z représente un phényle, éventuellement substitué par un ou plusieurs groupes choisis parmi un alkyle en C₁₋₈, un halogénoalkyle en C₁₋₈ ou un phényle ; un cycloalkyle en C₃₋₈, éventuellement substitué par un ou plusieurs groupes alkyle en C₁₋₈ ou un dialkyle en C₁₋₈-phosphinyle.

2. Procédé selon la revendication 1, dans lequel le catalyseur Ir-SpiroPAP est choisi parmi les composés IIIa ou IIIb, ou des énantiomères de ceux-ci,
dans lequel
R^{4a}, R^{4b}, R^{4c} et R^{4d} indépendamment les uns des autres représentent un hydrogène ou un alkyle en C₁₋₄ ;
le cycle en pointillé signifie un cycle aromatique lorsque Q¹ représente un azote et Q² représente un carbone et le cycle en pointillé signifie un cycle cycloalcane dans lequel Q¹ et Q² représentent un soufre ;
X¹ représente soit un ligand coordonné soit un contre-anion choisi parmi un halogène, un méthoxy, un tétrafluoroborate (BF4), un hexafluoroborate (BF6), un tétrakis(3,5-bis(trifluorométhyl)phényl)borate (barf), un acétylacétonate (acac), un hexafluorophosphate (PF6), un p-tolylsulfonate (OTs) ou un trifluorométhanesulfonate (OTf) et ;
Z représente un phényle, éventuellement substitué par un ou plusieurs groupes choisis parmi un alkyle en C₁₋₆, un halogénoalkyle en C₁₋₄ ou un phényle ou représente un cycloalkyle en C₄₋₇.

3. Procédé selon la revendication 1 ou 2, dans lequel le catalyseur Ir-SpiroPAP est choisi parmi les composés IIIa ou IIIb, ou des énantiomères de ceux-ci
R^{4a}, R^{4b}, R^{4c} et R^{4d} indépendamment les uns des autres représentent un hydrogène ou un alkyle en C₁₋₄ ;
le cycle en pointillé signifie un cycle aromatique lorsque Q¹ représente un azote et Q² représente un carbone et le cycle en pointillé signifie un cycle cycloalcane dans lequel Q¹ et Q² représentent un soufre ;
X¹ représente un halogène ;
Z représente un phényle, éventuellement substitué par un ou deux groupes choisis parmi un alkyle en C₁₋₆, un halogénoalkyle en C₁₋₄ ou un phényle ou représente un cyclopentyle ou un cyclohexyle.

4. Procédé selon l'une quelconque des revendications 1 à 3, dans lequel le catalyseur Ir-SpiroPAP est choisi parmi les composés 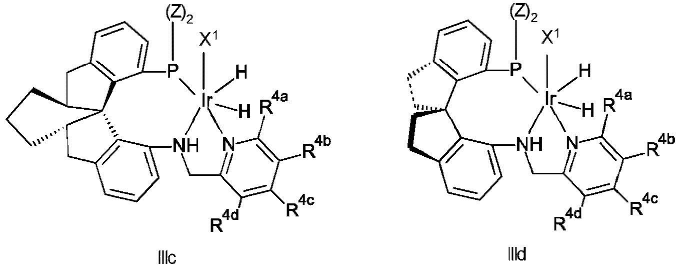 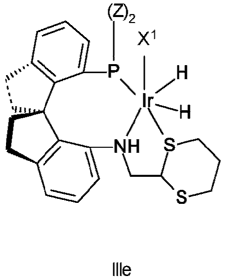 dans lequel ;
R^{4a}, R^{4b}, R^{4c} et R^{4d} indépendamment les uns des autres représentent un hydrogène ou un alkyle en C₁₋₄ ;
X¹ représente un halogène ;
Z représente un phényle éventuellement substitué par un ou deux groupes choisis parmi un alkyle en C₁₋₆, un halogénoalkyle en C₁₋₄ ou un phényle ou représente un cyclopentyle ou un cyclohexyle.

5. Procédé selon l'une quelconque des revendications 1 à 4, dans lequel l'hydrogénation asymétrique est réalisée en présence d'un solvant organique et d'une base à une pression d'hydrogène de 5 bar à 100 bar et à une température de réaction de 10 °C à 90 °C.

6. Procédé selon l'une quelconque des revendications 1 à 5, dans lequel le solvant organique est un alcool aliphatique, un alcool substitué par un halogène, un éther ou un solvant aromatique ou est un mélange de ceux-ci.

7. Procédé selon l'une quelconque des revendications 1 à 6, dans lequel la base est une base inorganique choisie parmi les carbonates ou hydrogénocarbonates ou phosphates ou hydrogénophosphates ou dihydrogénophosphates ou acétates ou formiates alcalins ou alcalino-terreux ou les bases organiques choisies parmi les amines, les alcoolates alcalins ou les amidines.

8. Procédé selon l'une quelconque des revendications 1 à 6, dans lequel le rapport substrat à catalyseur est choisi dans une plage de 100 à 10 000.

9. Procédé selon l'une quelconque des revendications 1 à 8, dans lequel le catalyseur Ir-SpiroPAP de formule IIIa ou IIIb est préparé in situ au cours de la réaction d'hydrogénation asymétrique en associant un complexe précatalytique d'iridium avec un ligand spiro-pyridylamidophosphine de formule 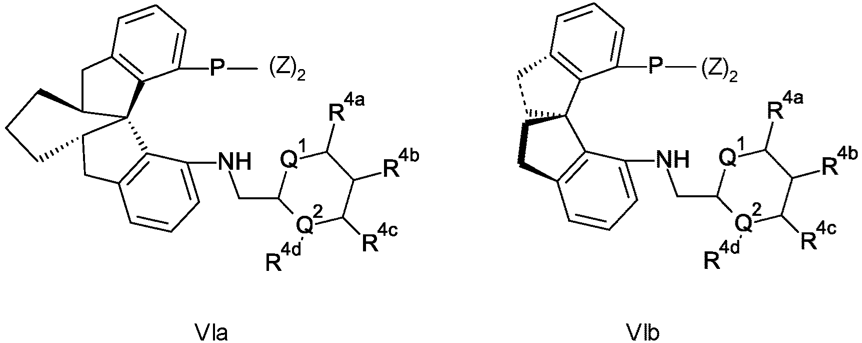 dans lequel R^{4a}, R^{4b}, R^{4c} et R^{4d}, Q¹ et Q² et Z ont les significations exposées ci-dessus.

10. Procédé selon la revendication 9, dans lequel le complexe précatalytique d'iridium est choisi parmi [Ir(cod)₂]BF₄, [IrCl(COD)]₂, [Ir(acac)(COD)], [Ir(OMe)(COD)]₂, [Ir(cod)₂]BARF, [Ir(cod)₂]PF6.

11. Procédé selon la revendication 1, dans lequel l'hydrogénation asymétrique de la cétone de formule IIa dans une première étape est réalisée en présence du catalyseur Ir-PEN de formule IVa ou IVb, ou d'énantiomères de celui-ci, 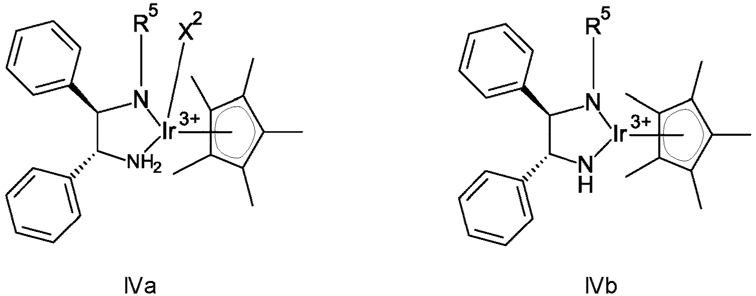 dans lequel,
R⁵ représente un alkyle en C₁₋₆-sulfonyle dans lequel le groupe alkyle est éventuellement substitué par un ou plusieurs atomes d'halogène ; avec un groupe 7,7-diméthyl-2-oxobicyclo[2.2.1]heptane-1-yle ou un phénylsulfonyle, dans lequel le groupe phényle est éventuellement substitué par un ou plusieurs groupes alkyle en C₁₋₆ et
X² représente soit un ligand coordonné soit un contre-anion choisi parmi un groupe alkyle en C₁₋₆-sulfonyloxy qui est éventuellement substitué par un ou plusieurs atomes d'halogène ; parmi un halogène, un alcoxy en C₁₋₆, un tétrahalogénoborate, un hexahalogénoborate, un tétrakis(3,5-bis(trihalogénoalkyle en C₁₋₆)phényl)borate, un acétylacétonate, une hexahalogénophosphine, un p-tolylsulfonate (OTs) ou un trihalogénométhanesulfonate ;
pour former la cétone de formule IIb, 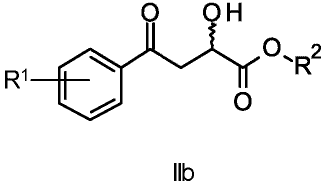 dans lequel R¹ et R² sont tels que ci-dessus,
et dans une étape ultérieure la cétone de formule IIb est en outre soumise à une hydrogénation asymétrique en présence d'un catalyseur Ir-SpiroPAP de formule IIIa ou IIIb, ou d'énantiomères de celui-ci, pour former le triol chiral de formule I.

12. Procédé selon la revendication 11, dans lequel
R⁵ représente un méthylsulfonyle, un trifluorométhylsulfonyle, un 7,7-diméthyl-2-oxobicyclo[2.2.1]heptan-1-yle ; un tolylsulfonyle ou un 1,3,5-tri-i-propylphénylsulfonyle ;
X² représente soit un ligand coordonné soit un contre-anion choisi parmi un groupe méthylsulfonyloxy qui est éventuellement substitué par un ou plusieurs atomes de fluor ; parmi un halogène, un méthoxy, un tétrafluoroborate (BF4), un hexafluoroborate (BF6), un tétrakis(3,5-bis(trifluorométhyl)phényl)borate (barf), un acétylacétonate (acac), une hexafluorophosphine (PF6), un p-tolylsulfonate (OTs) ou un trifluorométhanesulfonate (OTf).

13. Procédé selon la revendication 11 ou 12, dans lequel le catalyseur phénylènediamine d'iridium (catalyseur Ir-PEN) est de formule IVa, ou des énantiomères de celui-ci, dans lequel,
R⁵ représente un méthylsulfonyle, un trifluorométhylsulfonyle, un 7,7-diméthyl-2-oxobicyclo[2.2.1]heptane-1-yle ; un tolylsulfonyle ou un 1,3,5-tri-i-propylphénylsulfonyle ;
X² représente un groupe trifluorométhylsulfonyloxy ; ou
est de formule IVb, ou des énantiomères de celui-ci, dans lequel,
R⁵ représente un méthylsulfonyle, un trifluorométhylsulfonyle, un 7,7-diméthyl-2-oxobicyclo[2.2.1]heptane-1-yle ; un tolylsulfonyle ou un 1,3,5-tri-i-propylphénylsulfonyle.

14. Procédé selon l'une quelconque des revendications 11 à 13, dans lequel l'hydrogénation asymétrique de la cétone de formule IIa est réalisée en présence d'un solvant organique à une pression d'hydrogène de 5 bar à 100 bar et à une température de réaction de 10 °C à 90 °C.

15. Procédé selon la revendication 14, dans lequel le solvant organique est un alcool aliphatique, un alcool substitué par un halogène, un éther ou un solvant aromatique ou est un mélange de ceux-ci.

16. Procédé selon les revendications 14 ou 15, dans lequel le rapport substrat à catalyseur est choisi dans une plage de 100 à 1000.

17. Procédé selon l'une quelconque des revendications 1 à 16, dans lequel l'hydrogénation asymétrique de la cétone de formule IIa a lieu en présence d'un mélange d'un catalyseur Ir-Spiro PAP de formule IIIa ou IIIb, ou d'un énantiomère de celui-ci, et d'un catalyseur Ir-PEN de formule IVa ou IVb, ou d'un énantiomère de celui-ci.

18. Procédé selon la revendication 17 dans lequel la réaction est réalisée en présence d'un solvant organique et d'une base à une pression d'hydrogène de 5 bar à 100 bar et à une température de réaction de 10 °C à 90 °C.

19. Procédé selon les revendications 17 ou 18, dans lequel le solvant organique est un alcool aliphatique, un alcool substitué par un halogène, un éther ou un solvant aromatique ou est un mélange de ceux-ci.

20. Procédé selon l'une quelconque des revendications 17 à 19, dans lequel la base est une base inorganique choisie parmi les carbonates ou hydrogénocarbonates ou phosphates ou hydrogénophosphates ou dihydrogénophosphates ou acétates ou formiates alcalins ou alcalino-terreux ou les bases organiques choisies parmi les amines, les alcoolates alcalins ou les amidines.

21. Procédé selon l'une quelconque des revendications 17 à 20, dans lequel le rapport substrat au catalyseur Ir-PEN est choisi dans une plage de 100 à 10 000, de préférence dans une plage de 500 à 1000 et le rapport substrat au catalyseur Ir-Spiro PAP est choisi dans une plage de 100 à 10 000, de préférence dans une plage de 2500 à 7500.

22. Procédé selon la revendication 1, dans lequel les intermédiaires de l'hydrogénation asymétrique de la cétone de formule IIa en triol chiral de formule I de formule 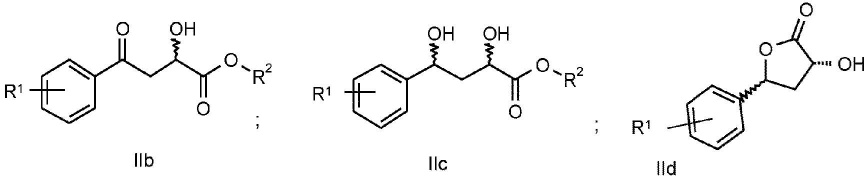 dans lequel R¹ et R² sont tels que ci-dessus, sont individuellement isolés et individuellement soumis à l'hydrogénation asymétrique en présence d'un catalyseur Ir-Spiro PAP de formule IIIa ou IIIb.

23. Procédé selon l'une quelconque des revendications 1 à 22, dans lequel le triol chiral a la formule Ia 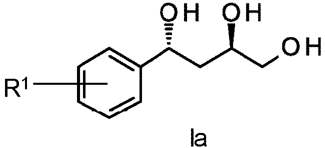 R¹ représente un hydrogène ou un halogène.

24. Procédé selon la revendication 23, dans lequel R¹ représente un halogène.

25. Procédé selon l'une quelconque des revendications 1 à 24, dans lequel le triol chiral a la formule Ib
